# EUROPÄISCHE PATENTSCHRIFT

(11) **EP 0 918 513 B1**
(45) Veröffentlichungstag und Bekanntmachung des Hinweises auf die Patenterteilung: **06.12.2000**
(21) Anmeldenummer: 97933611.2
(22) Anmeldetag: 14.08.1997
(51) Int. Cl.: A61K 9/50, A61K 9/54

(54) **GUT SCHLUCKBARE ORALE ARZNEIFORM**
EASY TO SWALLOW ORAL MEDICAMENT COMPOSITION
MEDICAMENT A ADMINISTRATION ORALE FACILE A AVALER

(30) Priorität: 15.08.1996 CH 200696
(43) Veröffentlichungstag der Anmeldung: 02.06.1999
(73) Patentinhaber: Losan Pharma GmbH, 79395 Neuenburg (DE)
(72) Erfinder: GRUBER, Peter, CH-4103 Bottmingen (CH)
(74) Vertreter: Zimmermann, Hans, Dr.
(86) Internationale Anmeldenummer: CH9700299
(87) Internationale Veröffentlichungsnummer: WO9806385

(56) Entgegenhaltungen:
- EP-A- 0 281 513
- EP-A- 0 662 320
- WO-A-88/06893
- WO-A-93/01800
- DATABASE WPI Week 9642 Derwent Publications Ltd., London, GB; AN 96-413087 XP002031496 & BR 9 403 617 A (RHODIA FARMA LTDA) , 3.Juni 1996 in der Anmeldung erwähnt

## Beschreibung

Die Erfindung betrifft eine gut schluckbare pharmazeutische Zusammensetzung zur oralen Verabreichung, enthaltend mindestens eine pharmazeutisch aktive Verbindung in wirksamer Menge und umfassend ein oder mehrere überzogene Teilchen, sowie ein Verfahren zu deren Herstellung und eine Methode zur Behandlung oder Verhütung von Krankheiten.

Die orale Einnahme fester Arzneiformen, wie Tabletten oder Kapseln, ist insbesondere in Abhängigkeit von ihrer Grösse mit Problemen behaftet, wobei vor allem bei Kindern und älteren Menschen oft Schluckbeschwerden auftreten können. Die gleichzeitige Verabreichung mehrerer Tabletten oder Kapseln kann zwar die Schluckbarkeit verbessern, beeinträchtigt aber andererseits die Patienten-Compliance. Um diese Probleme zu umgehen wurden bereits alternative Formulierungen vorgeschlagen, beispielsweise Pulver oder Granulate, die zu Suspensionen angerührt werden, Tabletten, die im Mund zerfallen, oder Kautabletten. Tabletten, die im Mund zerfallen, und Kautabletten sind aber für Wirkstoffe mit unangenehmem Geschmack oder für Arzneimittel mit verzögerter Wirkstofffreisetzung im allgemeinen wenig geeignet, da die zu diesem Zweck vorhandenen Überzüge durch den Tablettierprozess bzw. den Kauvorgang unvermeidlich beschädigt werden. Andererseits setzen sich im Falle von Suspensionen die Teilchen rasch ab und können beim Trinken meist nur unvollständig eingenommen werden, was zu unakzeptablen Abweichungen in der Dosierung führt. Durch Erhöhung der Viskosität kann dieses Problem nicht in befriedigender Weise behoben werden, weil eine entsprechend viskose Flüssigkeit nur ungern getrunken wird.

In EP-A-0 313 328 werden Brausetabletten oder wasserdispergierbare Tabletten mit verzögerter Wirkstofffreisetzung beschrieben, die durch Verpressen retardierter Teilchen mit einem Brausekörper oder anderen wasserdispergierbaren Ingredientien erhalten werden und in denen die retardierten Teilchen einen Überzug, enthaltend ein wasserquellbares Acrylpolymer und ein wasserlösliches hydroxyliertes Cellulosederivat, aufweisen. Beide Tablettenarten zerfallen in Wasser oder in der Mundhöhle und die freigesetzten Teilchen können zusammen mit den Hilfsstoffen geschluckt werden. Infolge des Zerfalls der Tablette können jedoch einzelne Teilchen im Glas oder in der Mundhöhle zurückbleiben, womit Abweichungen von der gewünschten Dosis auftreten können bzw. die Gefahr besteht, dass in der Mundhöhle schlecht schmeckende Wirkstoffe freigesetzt werden können. Ferner kann in Abhängigkeit des Anteils an wasserlöslichem hydroxyliertem Cellulosederivat die Freisetzungscharakteristik beeinträchtigt und der Wirkstoff zu rasch freigesetzt werden.

In ähnlicher Weise wird in EP-A-0 459 695 vorgeschlagen, Wirkstoffteilchen zur Geschmackskaschierung und/oder Verzögerung der Freisetzung mit einem Gemisch, umfassend Celluloseacetat und/oder Celluloseacetatbutyrat und Hydroxypropylcellulose, zu überziehen, wobei jedoch die überzogenen Teilchen vorzugsweise zu einer Kautablette verpresst werden. Diese haben aber - wie auch die vorangehend erwähnten Brausetabletten und wasserdispergierbaren Tabletten - den Nachteil, dass durch den Tablettierprozess die Retardhüllen oder geschmackskaschierenden Hüllen teilweise verletzt werden, womit die Freisetzungscharakteristik in unerwünschter Weise verändert wird bzw. in der Mundhöhle schlecht schmeckende Wirkstoffe freigesetzt werden können, was dazu führen kann, dass der Patient das Präparat ablehnt.

In WO-A-93/01800 wird hingegen empfohlen, ein Mikrogranulat aus einem biologischen Wirkstoff (insbesondere einem Protein) und einer schwachen Base nur teilweise mit einem Material zu überziehen, das die Freisetzung in der Mundhöhle und im Magen verzögert, und das teilweise überzogene Mikrogranulat mit einer Teilchengrösse von 50 bis 500 µm mit einem Säuerungmittel, das in Lösung einen pH-Wert von 1,5 bis 6 ergibt, und einem gelbildenden Mittel zu vermischen. Der bloss teilweise enterische Überzug soll zusammen mit der pH-Pufferung gewährleisten, dass eine Inaktivierung des biologischen Wirkstoffes vermieden und andererseits eine allmähliche Freisetzung im Dünndarm gewährleistet wird.

Über Pellets oder Granulatteilchen, die durch einen Überzug geschmackskaschiert werden, wird auch in WO-A-91/16043 berichtet. Die überzogenen Teilchen können zusammen mit einem Polymer (Xanthan-Gummi), Süssstoffen und Aromastoffen in Sachets abgefüllt werden. In ähnlicher Weise wird ferner in BR-A-9 403 617 empfohlen, Arzneimittel mit unangenehmem Geschmack in Form von Mikropellets mit einem natürlichen oder synthetischen Überzug herzustellen und diese mit Verdickungsmitteln, Süssstoffen, Aromastoffen und Farbstoffen zu mischen.

Demgegenüber wird in EP-A-0 662 320 ein Gemisch von bis zu 40 Gew.-% eines oral verabreichbaren Arzneimittels, mindestens 3 Gew.-% eines Geliermittels wie vorgelierte Stärke und bis zu 5 Gew.-% eines Bindemittels als trockene Gel-Zusammensetzung beschrieben, die beim Mischen mit der doppelten bis fünfzehnfachen Menge Wasser ein homogenes haferschleimartiges Gel ergeben soll. Im Falle von bitteren Wirkstoffen wird empfohlen, zusätzliche ein Maskierungsmittel wie Propylenglykol, Glycerin oder Polyethylenglykol oder einen Geschmacksverbesserer wie Kaliumglutamat oder Natriuminosinat zu verwenden oder - alternativ - den Wirkstoff zu pulverisieren und mit einem Maskierungsmittel zu überziehen.

In US-A-4 882 169 werden überzogene Pellets mit einem Durchmesser von 0,2 bis 3,0 mm vorgeschlagen, die in Wasser eine völlig homogene Dispersion bilden und die einen Kern, enthaltend Mikropartikel mindestens einer pharmazeutisch wirksamen Substanz, gegebenenfalls einen oder mehrere freigabesteuernde oder geschmacksmaskierende Überzüge und eine quellbare äussere Schicht aufweisen. Letztere enthält ein quellbares Polymer, vorzugsweise Guargummi. Da die quellbaren Materialien in Wasser rasch quellen und zerfallen, müssen sie mit einer Klebstofflösung besprüht werden, damit sie auf den Pellets haften. Die überzogenen Pellets werden zusammen mit Guargummi-Granulat und Aromastoffen in Sachets gefüllt, aus denen sie entnommen und in Wasser dispergiert werden können.

In US-A-5 288 500 wird ebenfalls vorgeschlagen, eine Vielzahl wirkstoffhaltiger Teilchen, die vorzugsweise mit einer diffusionskontrollierenden Schicht überzogen sein können und einen Durchmesser von 0,05 bis 7 mm aufweisen, mit einem Gelier- oder Quellmittel zu kombinieren. Letzteres kann im Gemisch mit den wirkstoffhaltigen Teilchen vorliegen, in einem Überzug enthalten sein, den wirkstoffhaltigen Teilchen vor dem Zumischen zu einem wässrigen Träger zugesetzt werden oder in einem wässrigen Träger, dem die wirkstoffhaltigen Teilchen zugemischt werden, dispergiert sein. Die vorgeschlagene Formulierung wird in einem wässrigen Träger dispergiert und bezweckt die Bildung einer glatten Oberfläche um jedes dispergierte Teilchen, womit Unebenheiten der Oberfläche der Teilchen maskiert und ein Kleben der Teilchen am Gefäss oder an der Mundschleimhaut verhindert werden soll.

Als Gelier- oder Quellmittel für die in US-A-5 288 500 vorgeschlagenen Formulierungen eignen sich Substanzen, insbesondere hydrophile Polymere, die in wässriger Umgebung kolloide Dispersionen, Sole oder Suspensionen bilden. Sie werden in einer Menge eingesetzt werden, die ausreicht, um die Unebenheiten der Teilchenoberflächen der dispergierten Teilchen zu maskieren, aber nicht so hoch ist, dass die Dispergierbarkeit in einem wässrigen Träger beeinträchtigt werden könnte. Gewünschtenfalls kann die Viskosität in unmittelbarer Nachbarschaft der dispergierten Teilchen durch Salzbildung, Chelatbildung, Veränderung der Polarität und dergleichen beeinflusst werden. Es soll jedoch vermieden werden, dass durch die gesamte Formulierung ein nahezu festes Gel gebildet wird. Die Komponenten der Formulierung können bis zur Verabreichung getrennt gehalten oder zusammen in Sachets abgefüllt oder zu Tabletten oder Kapseln verarbeitet werden; zur oralen Verabreichung werden sie zusammen in einem wässrigen Träger dispergiert.

Die in WO-A-91/16043, BR-A-9 403 617, EP-A-0 662 320, US-A-4 882 169 und US-A-5 228 500 beschriebenen Formulierungen werden zusammen mit einem wässrigen Träger eingenommen, in welchem sie vor der Verabreichung dispergiert werden. Hierbei zerfallen die Formulierungen in die Einzelteilchen, womit eine quantitative Einnahme in der Regel nicht mehr gewährleistet ist. Dies gilt selbst dann, wenn die dispergierten Teilchen - wie im Falle der in US-A-5 228 500 offenbarten Formulierungen - eine glatte Oberfläche aufweisen und nicht am Gefäss oder der Mundschleimhaut haften sollen. Generell stellt nämlich das Trinken einer Teilchensuspension oder -dispersion ein echtes Problem dar, weil nach dem Austrinken der Lösung in der Regel zumindest ein Teil der Teilchen am Boden des Gefässes zurückbleibt und nur mühsam in den Mund übergeführt werden kann und weil es in jedem Falle in hohem Masse unsicher ist, ob der Patient dies überhaupt versucht.

Zudem lassen sich Teilchensuspensionen oder -dispersionen in der Regel nicht quantitativ schlucken. Selbst wenn die Teilchen eine glatte, an der Mundschleimhaut nicht haftende Oberfläche aufweisen, kann im allgemeinen nicht verhindert werden, dass die Dosis nur unvollständig geschluckt wird und z.B. einzelne Teilchen zwischen den Zähnen hängen bleiben können. Dieses Problem ist - abgesehen von den unerwünschten Abweichungen von der vorgesehenen Dosierung - vor allem bei schlecht schmeckenden Wirkstoffen ein entscheidender Nachteil, da viele Wirkstoffe wie z.B. Ciprofloxazin, Loperamid, Cimetidin, Ranitidin, Acemetacin, Diclofenac-Natrium, Ibuprofen, Naproxen und Indomethacin so extrem bitter sind, dass eine quantitative Überführung der geschmackskaschierten Teilchen in den Magen unbedingt notwendig ist. Beispielsweise ergäbe ein einzelnes sich in der Mundhöhle auflösendes Ciprofloxacin-Teilchen bereits einen solch bitteren, schlechten Geschmack, dass das Präparat vom Patienten abgelehnt wird. Da aber andererseits das quellbare Polymer wegen der verlangten Wasserdispergierbarkeit zur Geschmacksmaskierung nur beschränkt geeignet ist, sind die vorbekannten Präparate für schlecht schmeckende Wirkstoffe nicht oder höchstens dann geeignet, wenn die Teilchen zusätzliche geschmacksmaskierende Überzüge aufweisen.

Die aus WO-A-91/16043, BR-A-9 403 617, EP-A-0 662 320, US-A-4 882 169 und US-A-5 288 500 bekannten Formulierungen sind somit mit dem Nachteil behaftet, dass sie für schlecht schmeckende Wirkstoffe kaum geeignet sind, dass eine quantitative Einnahme der vorgesehenen Dosis in der Regel nicht gewährleistet ist und dass sie in einem wässrigen Träger dispergiert werden müssen, d.h. sauberes Trinkwasser oder ein geeignetes Getränke zur Verfügung stehen muss, was in gewissen Gegenden oder z.B. auf Reisen die Einnahme erschweren oder verunmöglichen kann. Zudem müssen den vorbekannten Formulierungen meist Hilfsstoffe, z.B. ein Hilfsstoffgranulat, beigemischt werden; bekanntlich kann es aber hierbei, insbesondere bei Mischungen aus dichten Teilchen und Granulaten oder Mikropellets, zu Entmischungen kommen, was die Anwendung zusätzlich beeinträchtigen kann.

Methoden zur Geschmacksmaskierung von schlecht schmeckenden Wirkstoffen sind dem Fachmann auch aus anderen Quellen bekannt, wobei in der Regel die Wirkstoffe zwecks Verkleinerung der Oberfläche zu Pellets oder Granulaten verarbeitet und dann mit speichelresistenten Überzügen versehen werden.

Der vorliegenden Erfindung liegt die Aufgabe zugrunde, eine neue pharmazeutische Formulierung zur oralen Verabreichung bereitzustellen, die eine quantitative Einnahme der vorgesehenen Dosis gewährleistet, die Einnahme auch hoher Dosierungen ohne Schluckbeschwerden gestattet, den häufig unangenehmen Geschmack des Wirkstoffes kaschiert und vorzugsweise auch ohne Flüssigkeit eingenommen werden kann.

Die Aufgabe wird erfindungsgemäss gelöst durch eine pharmazeutische Zusammensetzung zur oralen Verabreichung, enthaltend mindestens einen pharmazeutischen Wirkstoff in wirksamer Menge und umfassend ein oder mehrere überzogene Teilchen, die einen Kern, enthaltend den mindestens einen pharmazeutischen Wirkstoff, und einen aus einer oder mehreren Schichten bestehenden Überzug aufweisen, wobei die Zusammensetzung dadurch gekennzeichnet ist, dass
(a) die Überzugsschicht bzw. die Überzugsschichten mindestens ein hydratisierbares, pharmazeutisch annehmbares Polymer enthalten, das bei Kontakt mit Speichel oder Wasser eine zusammenhängende, formbare, viskose, an der Oberfläche schlüpfrige, an der Mundschleimhaut nicht klebende Masse bildet, die den Austritt von wirkstoffhaltigen Teilchen aus der Masse und die Freisetzung von Wirkstoff in der Mundhöhle verhindert, und
(b) die Überzugsschicht bzw. die äusserste der Überzugsschichten eine wirksame Menge mindestens eines speichelflussfördernden Mittels enthält.

Im Gegensatz zu den vorbekannten Formulierungen wird somit die erfindungsgemässe Zusammensetzung nicht vor der Verabreichung in einem wässrigen Träger dispergiert oder suspendiert, sondern es wird - im Gegenteil - vermieden, dass die Zusammensetzung im Mund oder schon vor der Verabreichung in Einzelteilchen zerfällt. Es wurde nämlich gefunden, dass hydratisierbare Polymere in Gegenwart eines ausreichenden Speichelflusses rasch eine zusammenhängende, formbare, viskose Masse (d.h. eine plastische Masse) bilden, die den Austritt von Wirkstoffteilchen und die Freisetzung von Wirkstoffen in der Mundhöhle wirksam verhindern. Da die Masse bei Kontakt mit Speichel an der Oberfläche schlüpfrig wird, wird verhindert, dass die Zusammensetzung an der Mundschleimhaut oder an den Zähnen haftet. Durch die Bildung einer formbaren, viskosen Masse mit schlüpfriger Oberfläche wird somit das Schlucken der Zusammensetzung - auch im Falle hoher Dosierungen - erheblich erleichtert und erreicht, dass die Zusammensetzung leicht vom Mund durch den Gaumen in die Speiseröhre gleiten kann. Zudem wird durch das speichelflussfördernde Mittel ein ausreichend starker Speichelfluss ausgelöst, dass der gewünschte Effekt innert weniger Sekunden eintritt. Selbst bei hohen Dosierungen von bis zu 10 g wird in der Regel innert weniger als 20 Sekunden ein ausreichend formbarer, zusammenhängender, viskoser, plastischer "Teilchenbrei" gebildet, der das Schlucken der Zusammensetzung ohne Schwierigkeiten gestattet.

Die erfindungsgemässen Zusammensetzungen können somit im Unterschied zu den aus WO-A-91/16043, BR-A-9 403 617, EP-A-0 662 320, US-A-4 882 169 und US-A-5 288 500 bekannten Formulierungen durch direkte Applikation in den Mund und ohne vorherige Dispersion in einem wässrigen Träger verabreicht werden, womit vermieden wird, dass Teilchen in einem Gefäss oder in der Mundhöhle zurückbleiben können. Im Unterschied zu herkömmlichen Tablettenformulierungen können sie zudem vorzugsweise auch ohne zusätzliches Getränk eingenommen werden. Ferner können mit den erfindungsgemässen Formulierungen auch die Nachteile von Kautabletten vermieden werden. Es bereitet nämlich insbesondere Kindern und älteren Menschen oft Schwierigkeiten Kautabletten zu zerkauen und durch den Kauvorgang und die lange Verweilzeit im Mund erhöht sich die Gefahr der Wirkstofffreisetzung. Mit den erfindungsgemässen Formulierungen erübrigt sich ein Zerkauen und lange Verweilzeiten im Mund werden vermieden.

Die erfindungsgemässe Zusammensetzung kann wegen ihrer in Gegenwart von Speichel glatten, schlüpfrigen Oberfläche leicht vom Mund in die Speiseröhre und in den Magen gleiten und durch die Bildung einer zusammenhängenden, viskosen Masse wird ferner gewährleistet, dass die Zusammensetzung vollständig geschluckt werden kann, d.h. verhindert, dass einzelne Teilchen in der Mundhöhle oder zwischen den Zähnen zurückbleiben. Aufgrund der hohen Gleitfähigkeit können zudem selbst relativ grosse Teilchen, beispielsweise eine mit dem erfindungsgemässen Überzug versehene Tablette mit einem maximalen Durchmesser von bis zu 12 mm, in der Regel noch problemlos geschluckt werden.

Der erfindungsgemäss verwendete Überzug bietet zudem eine wirksamere und sicherere Geschmacksmaskierung als die herkömmlichen geschmacksmaskierenden Überzüge, da sie infolge der Bildung einer zusammenhängenden, viskosen Masse auch durch Verpressen der überzogenen Teilchen zu Tabletten nicht zerstört wird und andererseits die hohe Viskosität den Austritt von Wirkstoff wirksam verhindert.

Die erfindungsgemässe Zusammensetzung kann im wesentlichen wasserfrei sein oder, bezogen auf die wasserfreie Zusammensetzung, in der Regel bis zu etwa 300 Gew.-% oder mehr Wasser enthalten, ohne dabei in Einzelteilchen zu zerfallen oder in eine Dispersion oder Suspension überzugehen. Die Bezeichnung "im wesentlichen wasserfrei" bedeutet im Rahmen der vorliegenden Erfindung, dass der Zusammensetzung kein Wasser zugesetzt wird, dass sie aber handelsübliche Polymere und andere Hilfsstoffe enthalten kann, die oft einen geringen Prozentsatz an Wasser enthalten. Beispielsweise sind handelsübliche hydratisierbare Polymere, die häufig Wasser in Mengen von bis zu etwa 10 Gew.-% enthalten auch ohne zusätzliche Trocknung geeignet. Gewünschtenfalls können die erfindungsgemässen Formulierungen aber zusätzliches Wasser enthalten. Insbesondere kann eine im wesentlichen wasserfreie Zusammensetzung durch Zugabe von etwa 30 bis 300 Gew.-% Wasser, bezogen auf die wasserfreie Zusammensetzung, in eine weiche Arzneiform übergeführt werden, die die Einnahme hoher Dosierungen zusätzlich erleichtert und die eine nicht klebende Oberfläche und eine ausreichende Konsistenz aufweist, dass sie, ohne zu zerfallen, mit der Hand oder z.B. mittels eines Löffels oder Spatels eingenommen werden kann.

Die erfindungsgemässe Zusammensetzung eignet sich grundsätzlich zur Verabreichung beliebiger therapeutisch und/oder prophylaktisch wirksamer, oral verabreichbarer fester Wirkstoffe, beispielsweise Magen/Darmmittel und verdauungsfördernde Mittel wie Loperamid, Pektin, Plantagoovata-Samen, Leinsamen, Mesalazin (5-Aminosalicylsäure), Olsalazin, Cimetidin, Ranitidin, Famotidin, Nizatidin, Omeprazol, Sucralfat, Pantoprazol, Metoclopramid, Pancreatin, Amylase, Protease, Lipase und Sulfasalazin, Laxantien wie Trockenextrakt aus Sennesfrüchten, Faulbaumrinde oder -extrakt, Bisacodyl, Natriumpicosulfat und Lactulose, Analgetika und Antirheumatika wie Acetylsalicylsäure, Paracetamol, Ibuprofen, Morphin, Tramadol, Acemetacin, Propyphenazon, Naproxen, Diclofenac, Ketoprofen, Piroxicam, Meloxicam und Indomethacin, Antiallergika wie Dimetindenmaleat, Terfenadin, Astemizol und Ketotifen, Antitussiva und Expektorantia wie Ambroxol, Acetylcystein, Codein und Theophyllin, Betarezeptorenblocker, Calciumantagonisten und ACE-Hemmer wie Atenolol, Metoprolol, Pindolol, Nifedipin, Diltiazem, Verapamil, Captopril, Lisinopril und Enalapril, Koronarmittel wie Isosorbid, Isosorbidmononitrat und Molsidomin, Parkinsonmittel wie Levodopa, Psychopharmaka wie Amitriptylin, Trimipramin, Thioridazin, Oxazepam, Lorazepam und Piracetam, Sedativa wie Baldrian-Extrakt, Nitrazepam, Temazepam, Wirkstoffe wie Aescin, hochdosierte Aminosäuren, Budesonid, Furosemid und Pentoxifyllin, Vitamine wie Ascorbinsäure, Vitamin B₁, B₂, B₆ und/oder B₁₂, Folsäure und Vitamin E, Antibiotika wie Ciprofloxacin, Norfloxacin, Ofloxacin, Nalidixinsäure, Cinoxacin, Pefloxacin, Phenoxymethyl-Penicillin, Amoxicillin und weitere Penicilline mit einer Penam-Struktur, Cephalosporine mit einer Cephem-Struktur wie Cefaclor, Cefadroxil, Cefalexin, Cefpodoxim, Ceftibuten, Cefuroxim und Cefetamet, Clavulansäure in Kombination mit Amoxicillin, Tetracyciin, Makrolid-Antibiotika wie Erythromycin und seine Ester, Spiramycin und Josamycin, Colistin und Polymycin B, Nitrofurane wie Nitrofurantoin, Nitroimidazole wie Metronidazol, Sulfonamide wie Sulfadiazin und Sulfasalazin, und dergleichen sowie pharmazeutisch annehmbare Salze solcher Verbindungen, wie beispielsweise Olsalazin-Natrium, Pantoprazol-Natrium, Diclofenac-Natrium, Morphinsulfat, Codeinphosphat, Metoprololtartrat, Diltiazem-Hydrochlorid, Verapamil-Hydrochlorid, Amitriptylin-Hydrochlorid, Thioridazin-Hydrochlorid und dergleichen. Ferner eignet sich die erfindungsgemässe Zusammensetzung auch zur Verabreichung von Mineralsalzen wie Calciumlactat, Calciumcarbonat, Calciumgluconat, Magnesiumcarbonat, Magnesiumaspartat und Zinksulfat und von Spurenelementen. Gewünschtenfalls kann die Zusammensetzung Kombinationen von zwei oder mehreren Wirkstoffen enthalten.

Besonders bevorzugt sind Präparate, die als pharmazeutischen Wirkstoff Loperamid, Mesalazin, Olsalazin, Cimetidin, Ranitidin, Famotidin, Nizatidin, Omeprazol, Sucralfat, Pantoprazol, Pancreatin, Bisacodyl, Lactulose, Acetylsalicylsäure, Paracetamol, Ibuprofen, Morphin, Tramadol, Naproxen, Diclofenac, Piroxicam, Terfenadin, Astemizol, Ambroxol, Acetylcystein, Theophyllin, Atenolol, Nifedipin, Diltiazem, Verapamil, Isosorbidmononitrat, Amitriptylin, Nitrazepam, Budesonid, Ciprofloxacin, Norfloxacin, Ofloxacin, Amoxicillin, Cefaclor, Cefadroxil, Tetracyclin, Erythromycin, ein pharmazeutisch annehmbares Salz eines dieser Wirkstoffe oder eine Kombination zweier oder mehrerer dieser Wirkstoffe und Salze enthalten.

Als speichelflussfördernde Mittel eignen sich grundsätzlich beliebige pharmazeutisch annehmbare Substanzen, die einen starken Speichelfluss auslösen, vorzugsweise wasserlösliche organische Säuren, wie Weinsäure, Zitronensäure, Apfelsäure, Ascorbinsäure und dergleichen, und deren wasserlösliche Salze, insbesondere deren Natrium- und Kaliumsalze wie beispielsweise Natrium- oder Kaliumhydrogentartrat, Natriumhydrogencitrat oder Natriumascorbat, sowie wasserlösliche, osmotisch wirkende Substanzen, wie Glucose, Fructose, Saccharose, Xylit, Mannit, Sorbit, Maltit und dergleichen, wobei die erfindungsgemässen Zusammensetzungen mit Vorteil auch eine Kombination zweier oder mehrerer dieser Verbindungen enthalten können. Gemäss einer besonders bevorzugten Ausführungsform enthält die Überzugsschicht bzw. die äusserste der Überzugsschichten der erfindungsgemässen Zusammensetzung als speichelflussförderndes Mittel mindestens eine wasserlösliche organische Säure oder deren wasserlösliches Salz und mindestens eine wasserlösliche, osmotisch wirkende Substanz. Gewünschtenfalls kann die Überzugsschicht bzw. die äusserste der Überzugsschichten neben dem speichelflussfördernden Mittel Geschmackskorrigentien, wie Natriumchlorid, Aspartam, Natriumsaccharinat, Natriumcyclamat und dergleichen, enthalten, die die Wirkung des speichelflussfördernden Mittels im allgemeinen unterstützen.

Die erfindungsgemäss geeigneten hydratisierbaren Polymere sind solche, die in Gegenwart von Wasser oder Speichel rasch, vorzugsweise innert weniger als etwa 20 Sekunden, eine zusammenhängende, viskose Masse bilden können. Geeignete natürliche oder halbsynthetische Polymere sind dem Fachmann grundsätzlich bekannt. Besonders geeignet sind hydratisierbare Polymere, die in Wasser hochviskose Lösungen bilden, insbesondere nicht-ionische Polymere mit einer Viskosität, gemessen als 1 %-ige (Gew./Gew.) wässrige Lösung, von 3 bis 10000 mPa·s, beispielsweise Polyvinylpyrrolidon und Celluloseether wie Methylcellulose, Hydroxyethylcellulose, Hydroxypropylcellulose und Hydroxypropylmethylcellulose und dergleichen, und ionische Polymere mit einer Viskosität, gemessen als 1 %-ige (Gew./Gew.) wässrige Lösung, von 3 bis 30000 mPa·s, wie Natriumcarboxymethylcellulose, Polyacrylsäuren, Polyacrylate, Alginsäure, Alginate, Pektin, Xanthan, Galaktomannan, Guargummi, Hydroxypropylguargummi, Gelatine, Gummi arabicum und dergleichen, wobei solche mit einer Viskosität, gemessen als 1 %-ige (Gew./Gew.) wässrige Lösung, von mindestens etwa 25 mPa·s im allgemeinen besonders bevorzugt sind. Vorzugsweise kann der Überzug der erfindungsgemässen Zusammensetzungen auch zwei oder mehrere dieser Polymere enthalten.

Die im Rahmen der vorliegenden Erfindung verwendeten Viskositätswerte beziehen sich jeweils auf die Scherviskosität einer wässrigen Lösung bei 25°C. Gemessen wurde jeweils eine 1 %-ige (Gew./Gew.) wässrige Lösung, sofern nicht ausdrücklich etwas anderes angegeben ist. Die Messungen erfolgten mit einem Rotationsviskosimeter gemäss folgender Methode:

Das getrocknete Polymer wird unter Rühren mit einem mechanischen Rührwerk in der berechneten Menge Wasser gelöst. Die Lösung wird innert 30-60 Minuten mit einem Wasserbad auf eine Temperatur von 25°C ± 0,5°C eingestellt. Danach wird die Lösung ca. 10 Sekunden geschüttelt und dann sofort die Viskosität mittels eines Brookfield-Viskosimeters (Brookfield Synchro-Lectric Modell LVF mit vier Geschwindigkeiten und vier Spindeln und einem Messbereich von 0 bis 100000 mPa·s) gemessen, wobei die Spindel des Viskosimeters jeweils exakt 3 Minuten laufen gelassen, der Wert abgelesen und aus diesem durch Multiplikation mit dem für die gewählte Geschwindigkeit festgelegten Faktor die Viskosität der Lösung berechnet wird.

Beispiele besonders bevorzugter hydratisierbarer Polymere sind: Alginsäure oder Alginate, z.B. Natriumalginat, mit einer Viskosität von 25 bis 2000 mPa·s, einem pH von etwa 7 und einem Molekulargewicht von etwa 200000; Pektin mit einer Viskosität von 50 bis 5000 mPa·s, einem pH von 3,8 bis 5,2 und einem Molekulargewicht von etwa 100000; Xanthan mit einer Viskosität von 25 bis 3000 mPa-s, einem pH von etwa 6 und einem Molekulargewicht von über 1000000; Galaktomannan mit einer Viskosität von 50 bis 1000 mPa·s, einem pH von 4 bis 8 und einem Molekulargewicht von etwa 200000; Gelatine (Typ A oder B) mit einer Viskosität von 5 bis 200 mPa·s, einem pH von 4 bis 9 und einem Molekulargewicht von 10000 bis 100000; Natriumcarboxymethylcellulose mit einer Viskosität von 25 bis 8000 mPa·s, vorzugsweise 2500 bis 8000 mPa·s, mit einem Polymerisationsgrad von 500 bis 2000 und mit einem Substitutionsgrad von höchstens 3, d.h. höchstens 3, vorzugsweise 0,45 bis 1,45 und besonders bevorzugt 0,65 bis 0,95 Carboxymethylgruppen pro Anhydroglucoseeinheit; Celluloseether mit einer Viskosität von 3 bis 10000 mPa·s, einem Polymerisationsgrad von 40 bis 2000 und einem Substitutionsgrad von höchstens 3, inbesondere Hydroxyethylcellulose mit einer Viskosität von 3 bis 10000 mPa·s, vorzugsweise 25 bis 7000 mPa·s, und einem Substitutionsgrad von etwa 2,5, Hydroxypropylcellulose mit einer Viskosität von 10 bis 5000 mPa·s und einem Molekulargewicht von 80000 bis 1300000 und Methylhydroxypropylcellulose mit einer Viskosität von 3 bis 10000 mPa·s, mit einem Substitutionsgrad von 1 bis 2 und, vorzugsweise, einem Methyloxylgehalt von 18 bis 32% und einem Hydroxypropylgehalt von 7 bis 15%; Polyvinylpyrrolidon mit einem Molekulargewicht von 17000 bis 90000; Polyacrylsäuren und Polyacrylate mit einem Molekulargewicht von 400000 bis 4000000 und beispielsweise einer Viskosität von etwa 30000 mPa·s; wobei sich die Viskositäts- und pH-Werte jeweils auf eine 1 %-ige wässrige Lösung beziehen.

Während einige der hydratisierbaren Polymere, beispielsweise Polyvinylpyrrolidon oder Celluloseether, eine weitgehend pH-unabhängige Viskosität aufweisen, tritt bei anderen, beispielsweise bei Natriumcarboxymethylcellulose, Natriumalginat oder Polyacrylsäuren, im pH-Bereich des Magensaftes ein starker Viskositätsabbau auf. Dieser Unterschied kann daher ausgenützt werden, um den Zerfall der Zusammensetzung im Magen gezielt zu beeinflussen. Wird beispielsweise als hydratisierbares Polymer Natriumcarboxymethylcellulose, Natriumalginat oder eine Polyacrylsäure verwendet, wird einerseits im Mund die gewünschte hohe Viskosität erzielt, aber andererseits erreicht, dass die Zusammensetzung im Magen rasch zerfällt und damit eine rasche Wirkstofffreisetzung gestattet.

Die hydratisierbaren Polymere weisen vorzugweise eine mittlere Korngrösse von höchstens etwa 1,0 mm, typischerweise etwa 0,01 bis 1,0 mm, auf. Ist eine möglichst rasche und gleichmässige Bildung einer zusammenhängenden, viskosen Masse besonders wichtig, wird vorzugsweise eine kleine Korngrösse gewählt. In der Regel werden daher bevorzugt hydratisierbare Polymere mit einer mittleren Korngrösse von höchstens 200 µm, insbesondere höchstens 100 µm verwendet.

Die erfindungsgemässen Zusammensetzungen können gewünschtenfalls zwei oder mehrere, je mindestens ein hydratisierbares Polymer enthaltende Überzugsschichten aufweisen. Gemäss einer bevorzugten Ausführungsform der vorliegenden Erfindung kann der Überzug aus zwei oder mehreren Schichten bestehen, die dadurch gekennzeichnet sind, dass die als 1 %-ige (Gew./Gew.) wässrige Lösung gemessene Viskosität des hydratisierbaren Polymers einer Schicht jeweils nicht grösser ist und vorzugsweise kleiner sein kann als die als 1 %-ige (Gew./Gew.) wässrige Lösung gemessene Viskosität des hydratisierbaren Polymers der benachbarten inneren Schicht des Überzugs. Auf diese Weise können die geschmacksmaskierenden Eigenschaften zusätzlich verbessert und dennoch ein rasches Eindringen des Speichel gewährleistet werden, da der Speichel rasch in die äusserste Schicht eindringen kann und somit rasch eine zusammenhängende, viskose Masse mit schlüpfriger Oberfläche gebildet wird, während die innere Schicht bzw. die inneren Schichten zwar langsamer mit Speichel reagieren, aber einen hochviskosen Schleim erzeugen und damit eine äusserst wirksame Barriere gegen die Freisetzung von Wirkstoff bilden, was insbesondere im Falle von schlecht schmeckenden Wirkstoffen von Vorteil ist.

Vorzugsweise kann zu diesem Zweck die äusserste Schicht ein hydratisierbares Polymer mit einer Viskosität von 25 bis 5000 mPa·s enthalten und die zweitäusserste Schicht ein nicht-ionisches, hydratisierbares Polymer mit einer Viskosität von 5000 bis 10000 mPa·s und/oder ein ionisches Polymer mit einer Viskosität von 5000 bis 30000 mPa·s, wobei sich die Viskositätswerte jeweils auf eine 1 %-ige (Gew./Gew.) wässrige Lösung des Polymers beziehen.

Um eine möglich hohe Geschmacksmaskierung zu gewährleisten, wird in der zweitäussersten Schicht und in allfälligen weiteren inneren Schichten des Überzugs vorzugsweise ein hydratisierbares Polymer mit eine mittleren Korngrösse von höchstens 50 µm, beispielsweise 10-25 µm, verwendet werden. Die mittlere Korngrösse des hydratisierbaren Polymers der äussersten Schicht ist weniger kritisch, beträgt aber vorzugsweise nicht mehr als etas 200 µm.

Für die aus zwei oder mehreren Schichten bestehenden Überzüge eignen sich grundsätzlich alle oben genannten hydratisierbaren Polymere, soweit diese die verlangte Viskosität aufweisen. Vorzugsweise kann jedoch in der äussersten Schicht ein Polymer verwendet werden, das einen möglichst pH-unabhängigen Viskositätsaufbau gestattet. Als besonders geeignete hydratisierbare Polymere für die zweitäusserste und allfällige weitere innere Schichten des Überzugs haben sich beispielsweise Natriumcarboxymethylcellulose mit einer Viskosität von 5000 bis 8000 mPa·s, Polyacrylsäure mit einer Viskosität von 5000 bis 30000 mPa·s und Celluloseether mit einer Viskosität von 5000 bis 10000 mPa·s erwiesen, während sich für die äusserste Überzugsschicht vor allem nicht-ionische hydratisierbare Polymere, insbesondere Polyvinylpyrrolidon und Celluloseether mit einer Viskosität von 25 bis 5000 mPa·s bewährt haben (wobei sich die Viskositätswerte jeweils auf eine 1 %-ige (Gew./Gew.) wässrige Lösung des Polymers beziehen).

Gewünschtenfalls kann der Überzug auch aus einer einzigen Schicht bestehen, in welcher mindestens je eines der oben für die äusserste und die zweitäusserste Schicht angegebenen hydratisierbaren Polymer im Gemisch vorliegen. Auf diese Weise können ähnliche Vorteile, aber in geringerem Ausmass, erzielt werden.

Die Überzugsschicht bzw. Überzugsschichten können jeweils ein oder mehrere hydratisierbare Polymere, gewünschtenfalls viskositätsregulierende Hilfsstoffe wie Siliziumdioxid und ferner andere übliche Hilfsstoffe, beispielsweise Aromastoffe, Geschmackstoffe, Süssstoffe, pH-regulierende Hilfsstoffe, porenbildenden Hilfsstoffe wie Alkali- oder Erdalkalicarbonate oder -bicarbonate, oberflächenaktiven Hilfsstoffe, Tablettierhilfsstoffe wie mikrokristalline Cellulose, Maisstärke, Tablettensprengmittel und Schmiermittel (z.B. Stearinsäure oder Magnesiumstearat) und weitere Hilfsstoffe wie Stärke, Crospovidone, Croscarmelose, Talk und dergleichen enthalten, wobei Tablettierhilfsstoffe und Aroma-, Geschmacks- und Süssstoffe in der Regel nur in der äussersten Schicht eingesetzt werden, während andererseits die zweitäusserste Schicht und allfällige weitere innere Schichten vorzugsweise einen viskositätsregulierenden Hilfsstoff wie Siliciumdioxid und/oder einen basizitätserhöhenden Hilfsstoff wie Natriumhydrogencarbonat oder Natriumcarbonat enthalten können.

Der Anteil an hydratisierbarem Polymer in den Überzugsschichten kann in Abhängigkeit des verwendeten Polymers, der Verwendung von viskositätsregulierenden Hilfsstoffen, der gewünschten Verabreichungsform und dergleichen in gewissen Grenzen schwanken; er muss aber in jedem Fall ausreichend sein, dass bei Kontakt mit Speichel oder Wasser eine zusammenhängende, viskose Masse entsteht. Die optimalen Mengen können von Fall zu Fall durch Zugabe von Wasser oder Speichel leicht ermittelt werden. Typischerweise kann die Menge an hydratisierbarem Polymer in der zweitäussersten Schicht etwa 0,25 bis 35 Gew.-%, berechnet als im wesentlichen wasserfreies Polymer und bezogen auf den im wesentlichen wasserfreien wirkstoffhaltigen Kern, und in der äussersten Schicht etwa 1 bis 30 Gew.-%, berechnet als im wesentlichen wasserfreies Polymer und bezogen auf die im wesentlichen wasserfreie Gesamtzusammensetzung, betragen.

Der Anteil der Überzugsschicht bzw. Überzugsschichten in den erfindungsgemässen Zusammensetzungen kann vorzugsweise etwa 5 bis 75 Gew.-%, bezogen auf die im wesentlichen wasserfreie Zusammensetzung, betragen, entsprechend etwa 5 bis 300 Gew.-% an im wesentlichen wasserfreiem Überzug bezogen auf den wirkstoffhaltigen Kern. Besteht der Überzug aus zwei oder mehreren Schichten, kann der Anteil der zweitäussersten Schicht typischerweise etwa 0,25 bis 50 Gew.-%, insbesondere etwa 0,5 bis 20 Gew.-% und besonders bevorzugt etwa 1 bis 10 Gew.-%, berechnet als im wesentlichen wasserfreie Schicht und bezogen auf den im wesentlichen wasserfreien wirkstoffhaltigen Kern, und der Anteil der äussersten Schicht typischerweise etwa 3 bis 60 Gew.-%, berechnet als im wesentlichen wasserfreie Schicht und bezogen auf die im wesentlichen wasserfreie Zusammensetzung, betragen.

Die überzogenen Teilchen können typischerweise einen maximalen Durchmesser von etwa 0,25 bis 12 mm aufweisen und beispielsweise mit dem erfindungsgemässen Überzug versehene Granulate, Pellets oder Tabletten sein. Je nach gewünschter Applikationsart können diese überzogenen Teilchen entweder direkt verabreicht oder in an sich bekannter Weise mit üblichen Hilfsstoffen zu anderen oralen Verabreichungsformen weiterverarbeitet werden. Beispielsweise können überzogene Pellets oder Granulate mit üblichen Tablettierhilfsstoffen zu Tabletten verpresst werden, die mehrere oder eine Vielzahl von überzogenen Teilchen enthalten. Vorzugsweise können ferner mit einer oder mehreren inneren Überzugschichten versehene Pellets oder Granulate durch Zumischen des äusseren Schichtmaterials und z.B. durch Verpressen zur Tablette mit einer erfindungsgemässen äusseren Überzugsschicht umhüllt werden, d.h. eine Tablette gebildet werden, die mehrere überzogene Teilchen enthält und in der die äusserste Überzugsschicht zugleich als Tablettierhilfsmittel dient. Im Falle solcher Zusammensetzungen, die aus mehreren überzogenen Teilchen bestehen, wird - unabhängig davon, ob diese als Pellets oder Granulate vorliegen oder in einer Tablette enthalten sind - bei Kontakt mit Speichel jeweils rasch eine viskose Masse gebildet, die ein Zusammenkleben der Teilchen bewirkt. Weiterhin kann die erfindungsgemässe Zusammensetzung z.B. als Tablette ausgebildet sein, in der die Überzugsschicht bzw. die äusserste der Überzugsschichten nicht auf das wirkstoffhaltige Teilchen aufgetragen wurde, sondern dieses in Form einer Manteltablette umhüllt. Geeignete Applikationsformen und geeignete Weiterverarbeitungsmethoden sind dem Fachmann grundsätzlich geläufig.

Ferner kann die erfindungsgemässe Zusammensetzung beispielsweise aus einem einzigen überzogenen Teilchen mit einem maximalen Durchmesser von etwa 3 bis 12 mm bestehen und z.B. eine mit dem erfindungsgemässen Überzug versehene Tablette sein, da die hohe Gleitfähigkeit des Überzugs auch das Schlucken von Teilchen mit einem maximalen Durchmesser von mehr als 7 mm noch problemlos gestattet; vorzugsweise kann in diesem Fall der Überzug aus mindestens zwei Schichten bestehen.

Gemäss einer weiteren bevorzugten Applikationsform können im wesentlichen wasserfreie erfindungsgemässe Zusammensetzungen mit einer dosierten Menge von etwa 30 bis 300 Gew.-%, beispielsweise etwa 100 Gew.-% Wasser, bezogen auf die wasserfreie Zusammensetzung, versetzt werden, wobei das Wasser innert weniger Sekunden aufgesaugt wird und eine einzige, zusammenhängende, viskose Masse mit ausreichender Konsistenz gebildet wird, dass sie, ohne zu zerfallen, problemlos mit der Hand oder mittels eines Löffels oder Spatels entnommen werden kann. Die erhaltene "weiche" Formulierung weist somit einen Wassergehalt von etwa 23 bis 75 Gew.-%, beispielsweise 50 Gew.-%, bezogen auf die wasserhaltige Zusammensetzung, auf, und sie besitzt eine nicht klebende Oberfläche. Diese Applikationsart eignet sich insbesondere für die Einnahme mehrerer überzogener Teilchen, beispielsweise für die Einnahme einer dosierten Menge überzogener Pellets oder Granulate oder für die Einnahme einer mehrere überzogene Teilchen enthaltenden Tablette, und sie ermöglicht insbesondere die Einnahme sehr hoher Mengen von bis zu 10 g und mehr, ohne dass Schluckschwierigkeiten entstehen oder die Gefahr besteht, dass wirkstoffhaltige Teilchen in Mund zurückbleiben.

Die vorliegende Erfindung betrifft ebenfalls ein Verfahren zur Herstellung der neuen pharmazeutischen Zusammensetzung, welches dadurch gekennzeichnet ist, dass man ein oder mehrere Teilchen, enthaltend mindestens einen pharmazeutischen Wirkstoff in wirksamer Menge, mit einer oder mehreren Schichten überzieht, wobei
(a) die Schicht bzw. die Schichten mindestens ein hydratisierbares, pharmazeutisch annehmbares Polymer enthalten, das bei Kontakt mit Speichel oder Wasser eine zusammenhängende, formbare, viskose, an der Oberfläche schlüpfrige, an der Mundschleimhaut nicht klebende Masse bildet, die den Austritt von Wirkstoffteilchen aus der Masse und die Freisetzung von Wirkstoff in der Mundhöhle verhindert, und
(b) die Schicht bzw. die äusserste Schicht eine wirksame Menge mindestens eines speichelflussfördernden Mittels enthält,
dass man, gewünschtenfalls, die überzogenen Teilchen zusammen mit pharmazeutischen Hilfsstoffen in eine galenische Darreichungsform bringt und
dass man, gewünschtenfalls, die Zusammensetzung mit Wasser in einer Menge von bis zu etwa 300 Gew.-%, bezogen auf die im wesentlichen wasserfreie Zusammensetzung, versetzt.

Das Auftragen der Überzugsschichten auf die wirkstoffhaltigen Teilchen kann in an sich bekannter Weise nach gängigen Methoden erfolgen, beispielsweise durch abwechselndes Befeuchten und Beschichten mit einer Pulvermischung im Sphäronizer oder durch Aufsprühen einer Lösung oder Suspension der Schichtmaterialien. Diese und weitere geeignete Methoden sind dem Fachmann bestens geläufig. Wird der im wesentlichen wasserfreien Zusammensetzung Wasser zugesetzt, kann dies grundsätzlich in beliebigen Mengen bis zu etwa 300 Gew.-% erfolgen; vorzugsweise können jedoch etwa 30 bis 300 Gew.-%, bezogen auf die im wesentlichen wasserfreie Zusammensetzung, zugesetzt werden, um die oben erwähnte "weiche" Formulierung zu erhalten.

Die erfindungsgemäss verwendeten wirkstoffhaltigen Kerne können ebenfalls in an sich bekannter Weise nach üblichen Methoden erhalten werden. Gewünschtenfalls kann der Kern pH-regulierende Zusätze enthalten oder mit einer geschmackskaschierenden, magensaftresistenten oder die Wirkstofffreigabe verzögernden Lackschicht überzogen sein. Geeignete Materialien und Methoden sind dem Fachmann bestens bekannt.

Die vorliegende Erfindung betrifft ebenfalls eine Arzneimittelpackung, umfassend eine erfindungsgemässe pharmazeutische Zusammensetzung und die Anweisung, die Zusammensetzung durch direkte Applikation in den Mund einzunehmen oder vor der Einnahme mit einer Menge von 30 bis 300 Gew.-% Wasser, bezogen auf die im wesentlichen wasserfreie pharmazeutische Zusammensetzung, zu versetzen.

Gegenstand der Erfindung ist ferner eine Methode zur Behandlung oder Verhütung von Krankheiten durch orale Verabreichung einer pharmazeutischen Zusammensetzung, umfassend die Herstellung der erfindungsgemässen Zusammensetzung, gewünschtenfalls die Zugabe einer dosierten Menge von 30 bis 300 Gew.-% Wasser, bezogen auf die Zusammensetzung, und die direkte Applikation in den Mund, wobei vorzugsweise die Einnahme der Zusammensetzung auch ohne Einnahme einer Flüssigkeit erfolgen kann.

Weiterhin betrifft die vorliegende Erfindung die unter der Einwirkung von Speichel gebildete zusammenhängende, viskose Zusammensetzung, d.h. eine pharmazeutische Zusammensetzung zur oralen Verabreichung, umfassend ein oder mehrere Teilchen, die mindestens einen pharmazeutischen Wirkstoff in wirksamer Menge enthalten, und eine durch Kontakt mit Speichel gebildete, zusammenhängende, viskose, an der Oberfläche schlüpfrige, an der Mundschleimhaut nicht klebende Masse, die das wirkstoffhaltige Teilchen bzw. die wirkstoffhaltigen Teilchen umhüllt, die den Austritt von wirkstoffhaltigen Teilchen aus der Masse und die Freisetzung von Wirkstoff in der Mundhöhle verhindert und das Schlucken der Zusammensetzung erleichtert, und die eine wirksame Menge mindestens eines speichelflussfördernden Mittels und mindestens ein hydratisierbares, pharmazeutisch annehmbares Polymer in mindestens teilweise hydratisierter Form enthält.

Weitere bevorzugte Aspekte der erfindungsgemässen Zusammensetzung und deren Herstellung und Applikation ergeben sich aus den nachfolgenden Erläuterungen und den Ausführungsbeispielen.

Zur Herstellung der erfindungsgemässen Zusammensetzungen werden die Wirkstoffe im allgemeinen üblichen Hilfsstoffen zu Granulaten, Pellets, runden oder oblongen Tablettenkernen etc. mit einem Durchmesser von typischerweise etwa 0,1 bis 10 mm verarbeitet. Die Herstellung dieser wirkstoffhaltigen Teilchen kann in an sich bekannter Weise erfolgen.

Zur Herstellung von Granulaten mischt man beispielsweise den Wirkstoff mit Hilfsstoffen wie mikrokristalline Cellulose oder Lactose und befeuchtet die homogene Masse mit einer Bindemittellösung. Nach der Befeuchtung wird das Granulat gesiebt und getrocknet und eine staubfreie Fraktion von Teilchen mit einem Durchmesser von beispielsweise 0,1 bis 2,5 mm, vorzugsweise 0,25 bis 1,5 mm, abgesiebt.

Zur Herstellung von Pellets können alle gängigen Pellet-Herstellungsverfahren eingesetzt werden. Häufig geht man dabei von Starterkernen aus. Diese können beispielsweise mit Stärke ausgerundete Zuckerkügelchen, sphäroide Kristalle wie Saccharose, Weinsäure, Zitronensäure oder ähnliche, preiswerte und physiologisch unbedenkliche Hilfsstoffe sein und typischerweise einen Durchmesser von 0,1 bis 2 mm, vorzugsweise 0,4 bis 1,0 mm aufweisen. Nach der Befeuchtung mit einer Klebstofflösung wird der Wirkstoff als Pulver oder in gelöster oder suspendierter Form auf die Starterkerne aufgetragen. Im Falle von Wirkstofflösungen oder -suspensionen kann die Beschichtung der Starterkerne mit Vorteil in einem Wirbelschichtverfahren durch kontinuierliches Besprühen der Teilchen mit der Wirkstofflösung oder -suspension erfolgen. Bei der Pulverbeschichtung erfolgt die Beladung der Starterkerne zweckmässigerweise durch abwechselndes Befeuchten mit Klebstofflösung und Auftragen des Wirkstoffpulvers. Dem Wirkstoffpulver können gewünschtenfalls Hilfsstoffe wie Bindemittel, Siliziumdioxid oder Talk zugesetzt werden, um den Rundungsprozess optimal zu gestalten. Gewünschtenfalls kann die Klebstofflösung auch eine retardierende oder magensaftresistente Lacklösung oder -emulsion sein, um die Freisetzung des Wirkstoffes aus den beladenen Pellets zu beeinflussen. Gewünschtenfalls kann man den Wirkstoffpulvermischungen auch pH-regulierende, saure oder basische Hilfsstoffe zusetzen, die durch Schaffung eines entsprechenden Mikromilieus die Auflösung des Wirkstoffes beeinflussen. Geeigete Materialien und Methoden sind dem Fachmann bestens bekannt.

Die Herstellung der Pellets kann vorzugsweise in einem sogenannten Sphäronizer erfolgen. Hierbei werden die Starterkerne in einen mit einer drehenden Bodenplatte versehenen Kessel eingefüllt, in welchem sie auf der einen Seite mit einer Klebstoff- oder Polymerlösung besprüht und auf der gegenüberliegenden Seite über eine Dosierwaage mit dem Wirkstoffpulver bestäubt werden. In einem kontinuierlichen Prozess lässt sich auf diese Weise das Gewicht durch Beladung mit Pulver innert einer Stunde verdoppeln, wobei üblicherweise bis zu etwa 6 Teile Wirkstoffpulver auf 1 Teil Starterkerne aufgetragen werden können.

Eine weitere, für die erfindungsgemässen Zusammensetzungen geeignete Methode basiert auf der Extrusion und Sphäronisation. Hierbei werden die Wirkstoffe mit plastifizierenden Hilfsstoffen vermischt, befeuchtet und extrudiert. Das Extrudat mit einem Durchmesser von typischerweise etwa 0,4 bis 2 mm wird in den Sphäronizer übergeführt, wo es in zylinderförmige Teilchen zerbricht, die unter dem Einfluss der Zentrifugalkraft zu Kügelchen gerundet werden. Gewünschtenfalls kann das Freisetzungsverhalten der Wirkstoffpellets in bekannter Weise mit Polymerlösungen, retardierenden, lösungsvermittelnden oder pH-regulierenden Hilfsstoffen etc. beeinflusst werden.

Ferner können gewünschtenfalls Wirkstoffgranulate nach bekannten Methoden in Gummimatritzen zu Teilchen von etwa 1 mm isostatisch verpresst oder mit Spezialstempeln zu runden oder oblongen, etwa 1 bis 10 mm grossen Teilchen tablettiert werden.

Die erhaltenen wirkstoffhaltigen Teilchen können vor der Umhüllung mit dem erfindungsgemässen Überzug gewünschtenfalls mittels Besprühen mit Lack-Polymerlösungen in an sich bekannter Weise mit Diffusionsmembranen, erodierenden Membranen und/oder magensaftresistenten Membranen überzogen werden, womit die Freisetzungscharakteristik der erfindungsgemässen Zusammensetzungen in weiten Grenzen variiert und gegebenenfalls eine zusätzliche Geschmackmaskierung erzielt werden kann.

Die wirkstoffhaltigen Teilchen, die vorzugsweise einen maximalen Durchmesser von etwa 0,1 bis 10 mm, besonders bevorzugt etwa 0,25 bis 1,5 mm, aufweisen können, werden anschliessend mit einer oder mehreren, ein hydratisierbares Polymer enthaltenden Schichten überzogen. Die Auftragung der Schicht bzw. Schichten kann nach den üblichen Methoden und mit üblichen Geräten erfolgen, die dem Fachmann bestens bekannt sind. Bevorzugte Methoden und Anwendungsmöglichkeiten sind in Ausführungsbeispielen veranschaulicht und werden nachfolgend anhand einiger beispielhafter Formulierungen und Verabreichungsformen näher erläutert.

Im Falle des sehr bitter schmeckenden Loperamids, das der Patient bei starker Diarrhoe auch ohne Wasser überall einnehmen können sollte, kann eine erfindungsgemässe Formulierung beispielsweise dadurch erhalten werden, dass man Loperamid mit üblichen Hilfsstoffen zu einem 5 mm grossen Formling verpresst und den Formling in einer Beschichtungsapparatur mit Suspension aus Natriumalginat und Polyvinylpyrrolidon K 90 überzieht, wobei, bezogen auf das Gewicht des Formling, beispielsweise etwa 8 Gew.-% an Polymeren aufgetragen werden können. Anschliessend wird der überzogene Formling mit einer äusseren Polymerschicht aus einem niedriger viskosen Polymer, Zitronensäure, Aspartam und Zitronenaroma beschichtet.

In der Regel steht Reisenden in tropischen Ländern beim Auftreten einer starken Diarrhoe kein mikrobiologisch einwandfreies Wasser zur Einnahme einer Loperamid-Tablette oder -Kapsel zur Verfügung. Bei Einnahme der beschriebenen erfindungsgemässen Formulierung bewirkt jedoch die äussere Polymerschicht einen spontanen Speichelfluss in ausreichender Menge, der es ohne weiteres ermöglicht, den durch die hochviskose, zähe Gelschicht umhüllten Loperamid-Formling auch ohne Zuhilfenahme von Wasser zu schlucken. Da die Viskosität von Natriumalginat bei niedrigem pH stark abfällt, zerfällt die Tablette im Magen und der Wirkstoff wird rasch freigesetzt.

Im Falle von extrem schlecht schmeckenden, bitteren Wirkstoffen wie z.B. Ciprofloxacin können beispielsweise Wirkstoffgranulate oder -pellets zunächst mit einem geschmacksmaskierenden Überzug versehen werden. Hierzu können die Teilchen beispielsweise in bekannter Weise mit magensaftresistenten oder speichelresistenten Lacken überzogen oder vorzugsweise mit einer hydratisierbaren, hochviskosen, inneren Polymerschicht gemäss der vorliegenden Erfindung umhüllt werden. Als hydratisierbares Polymer eignet sich insbesondere mikronisierte Natriumcarboxymethylcellulose höchster Viskosität mit einer mittleren Korngrösse von etwa 10 bis 25 µm, die beispielsweise als Polymerpulver auf die mit einem Alkohol/Wasser-Gemisch befeuchteten Wirkstoffteilchen aufgetragen oder als Suspension in einem alkoholischen Lösungsmittel auf die Teilchen aufgesprüht werden kann. Dadurch erreicht man in einfacher Weise eine sehr gleichmässige Verteilung des Polymers auf den Wirkstoffteilchen, was für eine perfekte Speichelresistenz wichtig ist. Je nach gewünschter Geschwindigkeit der Wirkstofffreisetzung kann man, bezogen auf das Gewicht der Wirkstoffteilchen, beispielsweise etwa 0,5 bis 20 Gew.-%, vorzugsweise etwa 1 bis 10 Gew.-% an Polymer auftragen. Die überzogenen Wirkstoffteilchen werden anschliessend mit einer äusseren Polymerschicht überzogen, die mindestens ein niedriger viskoses, hydratisierbares Polymer, mindestens ein speichelflussförderndes Mittel und gegebenenfalls Hilfsstoffe wie Süssstoffe, Geschmacksstoffe, pH-regulierende, porenbildenden oder oberflächenaktive Hilfsstoffe und/oder Tablettierhilfsstoffe enthält, die das Eindringen des Speichels in die innere Polymerschicht erleichtern. Die äussere Polymerschicht kann vorzugsweise in einer Menge von etwa 5 bis 150 Gew.-%, bezogen auf das Gewicht der mit dem inneren Überzug versehenen Wirkstoffteilchen, aufgetragen werden.

Die Art und Menge des eingesetzten hydratisierbaren Polymers hängt bis zu einem gewissen Grad von der gewünschten Applikationsart ab. Werden beispielsweise überzogene Granulate oder Pellets z.B. mit Hilfe eines Löffels direkt in den Mund appliziert, so ist es wichtig, dass das Polymer die Teilchen innert weniger Sekunden zu einem konsistenten, zusammenhängenden, weichen "Teilchenbrei" zusammenklebt, wobei insbesondere bei extrem bitter schmeckenden Wirkstoffen vermieden werden muss, dass Teilchen in der Mundhöhle zurückbleiben können. Auf diese Weise können Mengen von z.B. 3 bis 4 g oder notfalls auch Mengen bis zu etwa 10 g noch problemlos und quantitativ geschluckt werden. Versucht man dagegen übliche retardierte Pellets in solchen Mengen zu schlucken, so ist es unvermeidlich, dass einige Pellets zwischen die Zähne oder in die Backentaschen geraten, so dass die Gefahr besteht, dass in der Mundhöhle allmählich Wirkstoff freigesetzt wird oder die Geschmacksmaskierung durch Kaubewegungen zerstört wird.

In einer weiteren, sehr vorteilhaften Applikationsform können erfindungsgemässe Pellets oder Granulate beispielsweise in einen Pelletspender, aus dem dosierte Mengen in einen kleinen Becher abgegeben werden können, gefüllt werden oder in tiefgezogene Näpfchen verpackt und mit einer Folie verschlossen werden. Geeignete Pelletspender und Verpackungsmaterialien sind dem Fachmann bestens bekannt. Vor der Verabreichung werden die erfindungsgemässen Teilchen im Becher bzw. Näpfchen z.B. mit Hilfe eines mitgelieferten Messlöffels mit einer dosierten Menge Wasser übergossen, wobei die Teilchen innert weniger Sekunden das zugefügte Wasser aufsaugen und in eine weiche, konsistente, zusammenhängende und die Wirkstoffteilchen umhüllende Masse übergehen, die an den Wänden des Bechers bzw. Näpfchens nicht klebt. Hierbei bleibt die Formulierung bei Zugabe von Mengen von bis zu 300 Gew.-% Wasser, bezogen auf das Gewicht der überzogenen Teilchen, in der Regel ausreichend konsistent, so dass sie mit der Hand oder mit Hilfe eines Spatels oder Löffels quantitativ entnommen werden kann. Appliziert man diese hydratisierte Formulierung auf die Zunge, so kann sie - unterstützt durch den eintretenden Speichelfluss - sehr leicht geschluckt werden.

Ein grosser Vorteil dieser Applikationsform besteht darin, dass auf diese Weise nahezu jede gewünschte Menge an Wirkstoff appliziert werden kann. Beispielsweise können Mengen von bis zu 10 g mit einem Wirkstoffgehalt von bis zu etwa 5 g geschluckt werden, ohne dass Schluckschwierigkeiten irgendwelcher Art auftreten. Überdies lässt sich die Masse unter der Einwirkung von Speichel mit der Zunge leicht in kleinere schluckfähige Klumpen formen, so dass gewünschtenfalls auch höhere Mengen problemlos eingenommen werden können. Ferner hat diese Applikationsform den Vorteil, dass die überzogenen Wirkstoffteilchen bereits vor der Einnahme zusammengeklebt werden und ein Wegdiffundieren von Wirkstoffteilchen in der Mundhöhle von vorneherein ausgeschlossen ist.

Erfindungsgemäss überzogene Teilchen wie Pellets oder Granulate können ferner zu Tabletten weiterverarbeitet werden. Hierzu können die Teilchen mit üblichen Tablettierhilfsstoffen gemischt und in an sich bekannter Weise zu Tabletten verpresst werden. Vorzugsweise können jedoch allfällige Tablettierhilfsstoffe im Überzug der Teilchen enthalten sein und die überzogenen Teilchen ohne Zusatz weiterer Hilfsstoffe direkt zu Tabletten verpresst werden, womit ein allfälliges Entmischen von Hilfsstoffen und Teilchen ausgeschlossen ist. Zur Herstellung von Tabletten, die eine Vielzahl von überzogenen Teilchen enthalten, werden daher vorzugsweise Teilchen wie Pellets oder Granulate verwendet, die einen aus mindestens zwei Schichten bestehenden Überzug aufweisen und die in der äussersten Überzugsschicht neben mindestens einem hydratisierbaren Polymer, mindestens einem speichelflussfördernden Mittel und allfälligen Hilfsstoffen, wie Süssstoffe und Aromen, vorzugsweise auch einen oder mehrere Tablettierhilfsstoffe wie mikrokristalline Cellulose und allenfalls weitere, das Eindringen von Speichel erleichternde Hilfsstoffe wie Stärke, Crospovidone, Croscarmelose und Siliziumdioxid enthalten können. Selbst bei geringem Pressdruck formen sich diese Teilchen zu einer gut zusammenhaltenden Tablette und infolge der leichten Verformbarkeit der äussersten Schicht wird die geschmackskaschierende Wirkung der inneren Schicht bzw. der inneren Schichten nicht zerstört. Bei Applikation in den Mund entsteht sofort eine verstärkter Speichelfluss und der Speichel dringt rasch in die Tablette ein und wandelt diese in einen weichen, zusammenhängenden, mit der Zunge formbaren "Teilchenbrei" um.

Wie bereits oben anhand der Loperamid-Formulierung illustiert, kann die erfindungsgemässe Zusammensetzung auch aus einem einzigen überzogenen Teilchen bestehen und beispielsweise eine mit dem erfindungsgemässen Überzug versehene Tablette sein. In diesen Fällen ist es im allgemeinen bevorzugt, einen aus mindestens zwei Schichten bestehenden Überzug mit den oben erläuterten Eigenschaften zu verwenden. Vorzugsweise kann eine solche Formulierung auch dadurch erhalten werden, dass die äusserste Schicht nicht auf das Teilchen aufgetragen wird, sondern dieses in Form einer Manteltablette umhüllt.

Die Erfindung wird durch die folgenden Beispiele weiter veranschaulicht. Viskositätsangaben beziehen sich jeweils auf eine 1 %-ige wässrige Lösung. Prozentangaben bezeichnen jeweils Gew.-%, sofern nicht ausdrücklich etwas anderes angegeben wird. Als hydratisierbare Polymere und Hilfsstoffe wurden in den Beispielen handelsübliche Materialien wie Polyvinylpyrrolidon K 25 oder Polyvinylpyrrolidon K 90 (BASF, Ludwigshafen/BRD), Crospovidone bzw. Polyplasdone (quervernetztes Polyvinylpyrrolidon; BASF, Ludwigshafen/BRD bzw. ISP/BRD), Croscarmelose (quervernetzte Natriumcarboxymethylcellulose; FMC, Brüssel/Belgien), Blanose 7H4 oder Blanose 7H9 (Natriumcarboxymethylcellulose; Aqualon, Düsseldorf/BRD), Culminal (Methylcellulose; Hercules, Milldate/USA), Natrosol 250 HHX (Hydroxyethylcellulose; Aqualon, Düsseldorf/BRD), Hydroxypropylcellulose LH 11 oder Hydroxypropylcellulose LH 20 (Shinetsu, Tokio/Japan), Klucel L (Hydroxypropylcellulose; Shinetsu, Tokio/Japan), Metolose 60 SH-50 (Hydroxypropylmethylcellulose; Shinetsu, Tokio/Japan), Pharmacoat (Hydroxypropylmethylcellulose; Shinetsu, Tokio/Japan), Tablettose (Lactose; Meggle, Wasserburg/BRD), Eudragit L- oder Eudragit S-Lack (Methacrylsäure-Polymer Typ A bzw. B; Röhm, Darmstadt/ BRD) und Eudragit RS-Lack (Ammoniummethacrylat-Copolymer Typ B; Röhm, Darmstadt/BRD) verwendet.

### Beispiel 1

| Herstellung des Wirkstoffkerns: | |
|---|---|
| Ciprofloxacin-Base, mikronisiert, oder Ciprofloxacin-Hydrochlorid | 2000 g |
| Crospovidone XL-M (Polyplasdone XL-M) | 110 g |
| Polyvinylpyrrolidon K 90 | 60 g |
| Wasser | 900 g |
| Ethanol | 1800 g |

Polyvinylpyrrolidon wird im Gemisch von Ethanol und Wasser gelöst; anschliessend werden die restlichen Pulver unter starkem Scheren in die Lösung eingerührt. Die 44,6 %-ige Suspension wird in einer Wirbelschichtapparatur bei einer Zulufttemperatur von 40°C auf 1,5 kg gesiebte Saccharose-Kristalle mit einem Durchmesser von 0,3 bis 0,6 mm aufgesprüht. Die getrockneten und gesiebten Teilchen (0,5-1,0 mm Durchmesser) enthalten ca. 54% Wirkstoff.

### Auftragen der Polymerschicht:

In einem Sphäronizer werden die Teilchen, jeweils nach vorherigem Befeuchten, portionenweise mit einer Pulvermischung aus
50 g Natriumchlorid (gemahlen),
50 g Natriumsaccharinat (gemahlen) und
50 g Natriumcarboxymethylstärke
beschichtet.
Danach werden die Teilchen, jeweils nach Befeuchten mit Ethanol/Wasser 1:1, mit einer Pulvermischung aus
275 g Natriumcarboxymethylcellulose (Blanose 7H9, 8000 mPa·s) und
75 g Talk
beschichtet.

### Beispiel 2

88 Gewichtsteile der im Beispiel 1 erhaltenen Pellets werden im Sphäronizer, jeweils nach vorherigem Befeuchten mit einer 10 %-igen ethanolischen Lösung von Polyvinylpyrrolidon K 25, mit 12 Gewichtsteilen einer Mischung aus

| | |
|---|---|
| Mannit | 4 Teile |
| Xanthan-Pulver (3000 mPa·s, MG 1000000) | 4 Teile |
| Mononatriumcitrat | 3 Teile |
| Natriumcyclamat/Natriumsaccharinat 9:1 | 0,25 Teile |
| Zitronen-Pulveraroma | 0,25 Teile |
| Siliziumdioxid | 0,5 Teile |

(Mengenangaben der Komponenten in Gewichtsteilen) beschichtet.

Danach wurden die Pellets zur Verbesserung der mechanischen Stabilität noch mittels einer Lösung, enthaltend 6 Gew.-% Hydroxypropymethylcellulose in Ethanol/Wasser 9:1, mit 0,5 Gew.-% (bezogen auf die eingesetzten Pellets) Hydroxypropylmethylcellulose überzogen.

Fügt man Wasser oder Speichel zu diesen Pellets bildet sich nahezu momentan eine klebende, zähviskose Schicht um die Pellets, die einerseits eine Diffusion des extrem bitter schmeckenden Wirkstoffes in die Mundhöhle für mindestens 3-5 Minuten verhindert und andererseits ein Zusammenkleben der dosierten Pellets zu einem zusammenhängenden, weichen, verformbaren Teilchenbrei bewirkt.

### Beispiel 3

Aus Ciprofloxacin-Hydrochlorid, mikrokristalliner Cellulose, Polyethylenglykol 6000 und Hydroxypropylcellulose LH 20 (Shin-Etsu, Japan) wird in an sich bekannter Weise eine plastifizierte Masse, enthaltend 80 Gew.-% Wirkstoff, hergestellt und durch Extrusion und Sphäronisation in Pellets mit einem Durchmesser von 0,6-1,25 mm übergeführt. 3 kg dieser Pellets werden im Sphäronizer, jeweils nach vorherigem Befeuchten mit einer 10 %-igen ethanolischen Polyvinylpyrrolidonlösung, mit 150 g gemahlenem Natriumcarbonat beschichtet. Anschliessend werden die Pellets in einer Filmcoatinganlage durch Besprühen mit einer 8 %-igen ethanolischen Lösung mit
50 g Ethylcellulose (7 mPa·s) und
25 g Talk
überzogen. Die erhaltenen Pellets sind wenigstens 5 Minuten speichelresistent und man verspürt während dieser Zeit praktisch keinen bitteren Geschmack im Mund.

3,1 kg der erhaltenen Pellets werden in einem Sphäronizer mittels einer Zweistoffdüse mit einer 25 %-igen ethanolischen Suspension von
70 g mikronisierter Natriumcarboxymethylcellulose (Blanose 7H4, 8000 mPa·s, mittlere Korngrösse 20 µm) und
23 g Hydroxypropylmethylcellulose (Metolose 60 SH-50, 25 mPa·s)
besprüht.

3,15 kg der erhaltenen, überzogenen Pellets werden im Sphäronizer jeweils nach vorherigem Befeuchten mit Ethanol/Wasser 1:1 mit einer Pulvermischung aus

| | |
|---|---|
| α-Lactosemonohydrat (200 mesh) | 200 g |
| Hydroxyethylcellulose (Natrosol 250 HHX, 3000 mPa·s) | 200 g |
| Mononatriumcitrat (Pulver) | 100 g |
| Natriumcyclamat/Natriumsaccharinat 9:1 | 12 g |
| Apfel-Trockenaroma | 11 g |
| Siliciumdioxid | 4 g |
| Hydroxypropylcellulose LH 11 | 15 g |

beschichtet. Vor dem Trocknen und Sieben werden die Pellets noch mit einer 6 %-igen Hydroxypropyimethylcellulose-Lösung (Ethanol/Wasser 8:2) überzogen bis zu einer Gewichtszunahme um 0,5%. 98% der Teilchen weisen einen maximalen Durchmesser zwischen 0,8 und 1,6 mm auf; der Wirkstoffgehalt beträgt 54%.

### Beispiel 4

In eine Hartgelatine-Kapsel der Grösse 000 (ohne Verschlussrille) füllt man 926 mg Pellets des Beispiels 3. Nach Öffnen der Kapsel durch Abziehen des Kapseloberteils entleert man die erfindungsgemässen Teilchen auf einen Teelöffel. Appliziert man diese Teilchen mittels des Löffels auf die Zunge, so entsteht ein spontaner Speichelfluss, der die Teilchen in wenigen Sekunden zu einem wohlschmeckenden Teilchenbrei zusammenklebt. Der gelartige Brei klebt weder an der Zunge noch an den Zähnen und kann leicht geschluckt werden.

Versetzt man die Teilchen auf dem Löffel mit ca. 1 ml Wasser, verschmelzen die Pellets innert 10 Sekunden zu einem viskosen, zusammenhängenden, an der Oberfläche nicht klebenden Brei. Dieser Teilchenbrei ist sehr leicht zu schlucken, ohne dass Teilchen in der Mundhöhle zurückbleiben.

### Beispiel 5

Je 1,85 g der nach Beispiel 3 erhaltenen Ciprofloxacin-Teilchen werden in runde, tiefgezogene PVC-Näpfe (PVC-Ausgangsfolie 250 µm) gefüllt und die Näpfe mit einer Peel-off-Folie verschlossen. Zur Applikation zieht man die Peel-off-Folie ab und fügt mit einem Applikatorlöffel 2 g Wasser in den tiefgezogenen Napf mit den Pellets. Das Wasser wird sofort aufgesaugt und die erfindungsgemässen Pellets gehen innert 10 Sekunden in einen viskosen, zusammenhängenden, an der Oberfläche nicht klebenden Pelletbrei über. Mit dem Spatelteil des Applikators lässt sich der zusammenhängende Brei quantitativ aus dem Napf entnehmen. Der an der Oberfläche gelartige Teilchenbrei lässt sich leicht quantitativ schlucken, ohne dass Teilchen in der Mundhöhle verbleiben.

### Beispiel 6

Je 1,85 g der nach Beispiel 3 erhaltenen Ciprofloxacin-Teilchen werden in runde, tiefgezogene, biplane Näpfe (Durchmesser 20 mm, Tiefe ca. 10 mm) gefüllt und die Näpfe mit Peel-off-Folie verschlossen. Die PVC-Ausgangsfolie hat eine Dicke von 150-200 µm. Zur Applikation zieht man die Peel-off-Folie ab und fügt mit dem Applikator 1,5 g Wasser zu den Ciprofloxacin-Teilchen. Das Wasser wird sofort aufgesaugt und die erfindungsgemässen Pellets bilden innert 30 Sekunden eine viskose, zusammenhängende, biplane Pelletbrei-Tablette. Drückt man von unten gegen den flexiblen Napf, so kann die Pelletbrei-Tablette quantitativ herausgedrückt werden. Sie ist an der Oberfläche gelartig und nicht klebend, und sie kann mit den Fingern aufgenommen und in den Mund gesteckt werden. Unter der Einwirkung des einsetzenden Speichelflusses kann die Tablette mit der Zunge zu einem oder zwei länglichen, gut schluckbaren Breistücken geformt werden.

### Beispiel 7

Ciprofloxacin-Pellets gemäss Beispiel 2 werden in einen Pelletspender gefüllt, der durch einmaliges Drehen des Dosierkopfes ca. 1,85 g Pellets in einen aufgesteckten Napf abgibt. Wie im Beispiel 5 beschrieben, kann mittels eines Applikators eine dosierte Menge Wasser in den Napf gegeben und der gebildete viskose zusammenhängende Pelletbrei mit einem rund geformten Spatel quantitativ entnommen werden.

### Beispiel 8

Zur Herstellung von Loperamid-haltigen Teilchen werden folgende Komponenten eingesetzt:

| | | pro Teilchen |
|---|---|---|
| Loperamid | 0,2 kg | 2 mg |
| Lactose | 5,0 kg | 50 mg |
| Polyvinylpyrrolidon | 0,2 kg | 2 mg |
| mikrokristalline Cellulose | 1,0 kg | 10 mg |
| Maisstärke | 0,6 kg | 6 mg |

wobei der mikronisierte Wirkstoff und die Hilfsstoffe gemischt und mit Wasser befeuchtet werden und die befeuchtete Masse durch ein 2,0 mm-Sieb gedrückt wird. Nach der Trocknung siebt man durch ein 0,8 mm-Sieb, fügt pro Teilchen 4,5 mg Crospovidone und 0,5 mg Magnesiumstearat hinzu und verpresst das Gemisch zu bikonvexen Teilchen mit einem Durchmesser von 5 mm.

Auf 6,0 kg der erhaltenen Teilchen (80000 Teilchen) werden 400 g Natriumalginat (2000 mPa·s, mittlere Korngrösse 15 µm) und 50 g Polyvinylpyrrolidon K 90 als Lösung bzw. Suspension in 1,2 kg Isopropanol aufgesprüht. Anschliessend wird auf die überzogenen Teilchen eine äussere Überzugsschicht aufgebracht, indem man die Teilchen mit einer Suspension aus
820 g Zitronensäure,
160 g Natriumsaccharinat,
25 g Zitronen-Flüssigaroma,
150 g Hydroxypropylcellulose (Klucel L, 20 mPa·s),
250 g Talk und
2645 g Ethanol (75 %-ig)
besprüht. Die Teilchen erzeugen nahezu momentan in der Mundhöhle einen beachtlichen Speichelfluss, mit dem ohne weiteres Wasser das mit einer zähviskosen, zusammenhängenden Schicht umhüllte Teilchen geschluckt werden kann. Der bittere Geschmack des Loperamids tritt nicht auf.

### Beispiel 9

3 kg Lactose-Granulatteilchen (Tablettose, 0,3-0,6 mm) werden in einer Beschichtungsapparatur mit einer Lösung aus
75 g Loperamid,
75 g Natriumsaccharinat,
150 g Natriumchlorid,
200 g Wasser und
800 g Ethanol
überzogen. Die erhaltenen Teilchen werden in einem Späronizer, jeweils nach vorherigem Befeuchten mit 10 %-iger ethanolischer Polyvinylpyrrolidon-Lösung, mit einem Gemisch aus
235 g Xanthan-Pulver (3000 mPa·s) und
50 g Talk
und anschliessend mit einem Gemisch aus
1,5 kg mikrokristalliner Cellulose,
0,2 kg Zitronensäure (Pulver),
0,1 kg Aspartam,
0,1 kg Orangen-Trockenaroma und
150 g Methylcellulose (Culminal, 3000 mPa·s)
überzogen. Die getrockneten Teilchen werden zu biplanen Tabletten mit einem Gewicht von 150 mg (8 mm Durchmesser) verpresst. Innert 20 Sekunden ergeben die Tabletten im Mund infolge des angeregten Speichelflusses einen zusammenhängenden, weichen, viskosen Teilchenbrei, der ohne Wasser sehr leicht zu schlucken ist. Der bittere Geschmack des Loperamids ist nicht festzustellen.

### Beispiel 10

Auf 3,0 kg gemäss Beispiel 3 hergestellter, mit einer Schicht aus Natriumcarboxymethylcellulose und Hydroxypropylmethylcellulose überzogener Ciprofloxacinhydrochlorid-Teilchen (ohne speichelflussfördernde äussere Schicht) wird, jeweils nach vorherigem Befeuchten mit einer 10 %-igen ethanolischen Polyvinylpyrrolidon-Lösung (Verbrauch: 1,3 kg Lösung), portionenweise ein Gemisch aus
1,7 kg mikrokristalline Cellulose,
0,3 kg Natriumcarboxymethylstärke,
0,15 kg Zitronensäure,
0,4 kg Hydroxypropylmethylcellulose (7,5 mPa·s),
0,05 kg Aspartam und
0,1 kg Zitronenaroma-Trockenpulver.
aufgetragen. Nach dem Trocknen haben die Teilchen einen Gehalt von ca. 33 Gew.-% Ciprofloxacin-Hydrochlorid.

Aus diesen Teilchen werden nach dem Zumischen von 0,5 Gew.-% Magnesiumstearat bei geringem Druck biplane Tabletten mit einem Durchmesser von 20 mm und einem Wirkstoffgehalt von 750 mg Ciprofloxacin-Hydrochlorid gepresst. Die Härte der Tabletten beträgt ca. 60 N. Legt man eine Tablette auf die Zunge, bewirkt der einsetzende Speichelfluss innert 20 Sekunden die Bildung eines zusammenhängenden, viskosen Teilchenbreis, der an der Zunge, dem Gaumen und den Zähnen nicht klebt und der leicht geschluckt werden kann.

### Beispiel 11

3 kg in an sich bekannter Weise hergestellte, magensaftresistente Pellets mit Wirkstoffgehalt von 73 Gew.-% 5-Aminosalicylsäure werden in einem Sphäronizer, jeweils nach vorherigem Besprühen mit einer 10 %-igen Polyvinylpyrrolidon-Lösung in Ethanol/Wasser 7:3, mit einem Pulvergemisch aus
100 g Natriumcarboxymethylcellulose (Blanose 7H4, 8000 mPa·s, mittlere Korngrösse 20 µm),
25 g Mononatriumcitrat,
15 g Aspartam und
30 g Vanillin-Trockenpulver
beschichtet. Die getrockneten und gesiebten Teilchen (0,8-1,4 mm) werden in einen Pelletspender gefüllt, der durch Drehen des Dosierkopfes 1450 mg Pellets, entsprechend 1000 mg 5-Aminosalicylsäure, abgibt. Werden die Pellets mit einem Löffel auf die Zunge plaziert, ergeben sie innert 10 Sekunden einen weichen, zusammenhängenden Pelletbrei, der sich leicht schlucken lässt.

### Beispiel 12

3 kg in an sich bekannter Weise hergestellte, magensaftresistente Pellets mit Wirkstoffgehalt von 73 Gew.-% 5-Aminosalicylsäure werden in einem Sphäronizer, jeweils nach vorherigem Besprühen mit einer 10 %-igen ethanolischen Polyvinylpyrrolidon-Lösung, portionenweise mit einer Pulvermischung aus
1,6 kg mikrokristalliner Cellulose,
0,3 kg Natriumcarboxymethylstärke,
0,3 kg Adipinsäure,
0,13 kg Natriumhydrogencarbonat,
0,15 kg Hydroxypropylmethylcellulose (Pharmacoat, 25 mPa·s),
0,12 kg Aspartam,
0,11 kg Vanillin-Trockenpulver und
0,1 kg Siliziumdioxid
beschichtet. Zur Beschichtung benötigt man 1,5 kg Polyvinylpyrrolidon-Lösung. Nach der Trocknung haben die Teilchen einen Wirkstoffgehalt von ca. 35 Gew.-%. Aus diesen Teilchen werden bei geringem Druck biplane Tabletten mit einem Durchmesser von 23 mm und einem Gewicht von 2860 mg, entsprechend 1000 mg Wirkstoff, gepresst. Die Härte der Tabletten beträgt 60-80 N. Die Tabletten erzeugen im Mund einen erhöhten Speichelfluss und bilden innert 20 Sekunden einen zusammenhaftenden, weichen, mit der Zunge leicht verformbaren Pelletbrei, der sich leicht schlucken lässt.

### Beispiel 13

Aus Amoxicillin, mikrokristalliner Cellulose, Hydroxypropylcellulose und Lactose werden nach bekannten Verfahren der Extrusion und Sphäronisation Wirkstoffpellets mit einem Amoxicillingehalt von 68 Gew.-% hergestellt. Ein Teil dieser Pellets wird in bekannter Weise durch Überziehen mit Eudragit L und Eurdragit RS in freigabeverzögerte, magensaftresistente Pellets übergeführt.

3 kg dieser freigabeverzögerten Pellets (Wirkstoffgehalt 59,1 Gew.-%) und 3 kg nicht verzögerter Pellets (Wirkstoffgehalt 68 Gew.-%) werden in einem Sphäronizer vorsichtig mit Wasser besprüht und anschliessend mit einer Pulvermischung aus
60 g Talk und
60 g Natriumcarboxymethylcellulose (Blanose 7H4, 8000 mPa·s, mittlere Korngrösse 20 µm)
beschichtet. Danach werden die Teilchen im Luftstrom und unter Rotieren der Sphäronizer-Scheibe langsam mit einer Suspension folgender Zusammensetzung übergossen:
30 g Aspartam,
45 g Zitronensäure,
50 g Vanillin-Trockenaroma,
150 g Hydroxyethylcellulose (Teilchengrösse 25 µm, 4500 mPa·s),
75 g Talk,
75 g Polyvinylpyrrolidon K 25 und
650 g Ethanol.

Die erhaltenen Pellets, die einen Wirkstoffgehalt von ca. 58 Gew.-% aufweisen, werden in einen Pelletspender gefüllt, der bei einer halben Umdrehung des Dosierkopfes ca. 860 mg und bei einer vollen Umdrehung ca. 1720 mg auf z.B. einen Löffel dosiert (entsprechend 500 bzs. 1000 mg Amoxicillin). Werden die Pellets mit dem Löffel auf die Zunge plaziert, erzeugen sie einen spontanen Speichelfluss und gehen in einen weichen, fruchtig schmeckenden Pelletbrei über, der sich leicht schlucken lässt.

### Beispiel 14

Ausgesiebte Ascorbinsäurekristalle werden in einer Wirbelschichtapparatur mit einer konzentrierten wässrigen Ascorbinsäure-Lösung ausgerundet. Nach dem Trocknen wird die Ascorbinsäure-Fraktion mit einer Teilchengrösse von 0,315 bis 0,7 mm (10 kg) in einem Sphäronizer mit einer Suspension nachfolgender Zusammensetzung langsam übergossen und im Luftstrom getrocknet:
150 g Aspartam,
150 g Zitronen-Aroma,
250 g Methylhydroxypropylcellulose (4500 mPa·s),
50 g Talk,
50 g Polyvinylpyrrolidon K 25 und
1400 g Ethanol.

Das gesiebte und getrocknete Material wird in einen Pelletspender gefüllt, dessen Dosierkammern so eingestellt sind, dass bei einer Umdrehung um 180° eine 500 mg Ascorbinsäure enthaltende Menge Pellets (532 mg) dosiert wird. Gibt man diese auf die Zunge, so entsteht durch spontanen Speichelfluss ein nach Zitrone schmeckender, zusammenhängender, sauer-süsser Pelletbrei, der leicht heruntergeschluckt werden kann.

### Beispiel 15

In einem Vakuum-Feuchtmischgerät werden 10 kg Zitronensäurekristalle mit einer Teilchengrösse von 0,25 bis 0,6 mm vorgelegt. Die Kristalle werden nach Befeuchten mit Ethanol so lange gerührt, bis eine klebrige Masse entsteht. Diese wird mit Calciumcarbonat-Pulver abgestreut, wobei sich das Calciumcarbonat vollständig auf die Zitronensäurekristalle bindet. Nach erneutem Befeuchten mit Ethanol streut man wieder mit Calciumcarbonat ab. Auf diese Weise entstehen schliesslich gut ausgerundete Calciumcarbonat-Pellets mit einem Zitronensäurekern. Insgesamt werden nach diesem Verfahren 5,8 kg Calciumcarbonat aufgetragen.

In der gleichen Apparatur werden 15 kg gesiebte Calciumcarbonat-Zitronensäure-Pellets (Fraktion 0,4-1,0 mm) unter Rühren mit einer Suspension aus
200 g Zitronensäure,
150 g Aspartam,
150 g Zitronen-Aroma,
50 g Orangen-Aroma,
450 g Natriumcarboxymethylcellulose (Blanose 7H4, 8000 mPa·s, mittlere Korngrösse 20 µm),
200 g Talk und
1900 g Ethanol
im Vakuum beschichtet und gleichzeitig getrocknet.

Die erhaltenen Pellets können z.B. in einen Pelletspender plaziert werden, dessen Dosierkammern so eingestellt sind, dass durch eine Umdrehung um 360° 2,350 g Calciumcarbonat-Pellets dosiert werden, die die empfohlene Tagesdosis von 800 mg Calcium enthalten. Werden die Pellets z.B. mit Hilfe eines Löffels auf der Zunge plaziert, entsteht durch den rasch einsetzenden Speichelfluss eine weiche Pelletmasse, die sehr leicht heruntergeschluckt werden kann.

In analoger Weise können unter Verwendung von Magnesiumcarbonat anstelle von Calciumcarbonat entsprechende Magnesiumcarbonat-Pellets erhalten und diese z.B. in einen Pelletspender plaziert werden, der bei einer halben Umdrehung 1,25 g Pellets dosiert, die 5 mMol (120 mg) Magnesium enthalten.

### Beispiel 16

In analoger Weise zu Beispiel 15 werden in einem Vakuumgranulierer 14 kg Zitronensäurekristalle beschichtet, aber anstelle von Calciumcarbonat mit 1,4 kg Vitaminmischung, enthaltend die Vitamine B₁, B₂, B₆, B₁₂, Folsäure, Vitamin E, Biotin, Vitamin C, Pantothensäure, Niacin und als Trägerstoff Maltodextrin. 200 mg der Vitaminmischung enthalten den Tagesbedarf der genannten Vitamine.

Ohne Siebung werden die beschichteten Zitronensäure-Vitamin-Pellets in der gleichen Apparatur mit einer Suspension aus
60 g Weinsäure,
70 g Natriumcyclamat,
70 g Orangen-Aroma,
70 g Talk,
100 g Polyvinylpyrrolidon K 25,
400 g Methylhydroxypropylcellulose (Methocel E 6, 6 mPa·s) und
2100 g Ethanol
beschichtet und im Vakuum getrocknet.

Die beschichteten Pellets können z.B. in einen Pelletspender plaziert werden, dessen Dosierkammern so eingestellt sind, dass bei einer Umdrehung um 360° 2,34 g Pellets dosiert werden, die die empfohlene Tagesdosis an den genannten Vitaminen enthalten. Werden die Pellets mit einem Löffel auf der Zunge plaziert, ergeben sie innert 10 Sekunden einen weichen, zusammenhängenden Pelletbrei, der fruchtig nach Orangen schmeckt und sich leicht schlucken lässt.

## Patentansprüche

1. Pharmazeutische Zusammensetzung zur oralen Verabreichung, enthaltend mindestens einen pharmazeutischen Wirkstoff in wirksamer Menge und umfassend ein oder mehrere überzogene Teilchen, die einen Kern, enthaltend den mindestens einen pharmazeutischen Wirkstoff, und einen aus einer oder mehreren Schichten bestehenden Überzug aufweisen, dadurch gekennzeichnet, dass
(a) die Überzugschicht bzw. die Überzugsschichten mindestens ein hydratisierbares, pharmazeutisch annehmbares Polymer enthalten, das bei Kontakt mit Speichel oder Wasser eine zusammenhängende, formbare, viskose, plastische, an der Oberfläche schlüpfrige, an der Mundschleimhaut nicht klebende Masse bildet, die das wirkstoffhaltige Teilchen bzw. die wirkstoffhaltigen Teilchen umhüllt und die den Austritt von wirkstoffhaltigen Teilchen aus der Masse und die Freisetzung von Wirkstoff in der Mundhöhle verhindert, und
(b) die Überzugsschicht bzw. die äusserste der Überzugsschichten eine wirksame Menge mindestens eines speichelflussfördernden Mittels enthält.

2. Zusammensetzung nach Anspruch 1, dadurch gekennzeichnet, dass sie als hydratisierbares Polymer ein nicht-ionisches Polymer mit einer Viskosität, gemessen als 1 %-ige (Gew./Gew.) wässrige Lösung bei 25°C, von 3 bis 10000 mPa·s oder ein ionisches Polymer mit einer Viskosität, gemessen als 1 %-ige (Gew./Gew.) wässrige Lösung bei 25°C, von 3 bis 30000 mPa·s, enthält.

3. Zusammensetzung nach Anspruch 1 oder 2, dadurch gekennzeichnet, dass sie als hydratisierbares Polymer Methylcellulose, Hydroxyethylcellulose, Hydroxypropylcellulose, Hydroxypropylmethylcellulose, Polyvinylpyrrolidon, Natriumcarboxymethylcellulose, Polyacrylsäure, Polyacrylat, Alginsäure, Alginat, Pektin, Xanthan, Galaktomannan, Guargummi, Hydroxypropylguargummi, Gelatine und/oder Gummi arabicum enthält.

4. Zusammensetzung nach einem der Ansprüche 1 bis 3, dadurch gekennzeichnet, dass sie ein hydratisierbares Polymer mit einer Viskosität, gemessen als 1 %-ige (Gew./Gew.) wässrige Lösung bei 25°C, von mindestens etwa 25 mPa·s enthält.

5. Zusammensetzung nach einem der Ansprüche 1 bis 4, dadurch gekennzeichnet, dass das hydratisierbare Polymer eine mittlere Korngrösse von höchstens 200 µm aufweist.

6. Zusammensetzung nach einem der Ansprüche 1 bis 5, dadurch gekennzeichnet, dass der Überzug in einer Menge von 5 bis 75 Gew.-%, bezogen auf die im wesentlichen wasserfreie Zusammensetzung, vorliegt.

7. Zusammensetzung nach einem der Ansprüche 1 bis 6, dadurch gekennzeichnet, dass sie als pharmazeutischen Wirkstoff Loperamid, Mesalazin, Olsalazin, Cimetidin, Ranitidin, Famotidin, Nizatidin, Omeprazol, Sucralfat, Pantoprazol, Pancreatin, Bisacodyl, Lactulose, Acetylsalicylsäure, Paracetamol, Ibuprofen, Morphin, Tramadol, Naproxen, Diclofenac, Piroxicam, Terfenadin, Astemizol, Ambroxol, Acetylcystein, Theophyllin, Atenolol, Nifedipin, Diltiazem, Verapamil, Isosorbidmononitrat, Amitriptylin, Nitrazepam, Budesonid, Ciprofloxacin, Norfloxacin, Ofloxacin, Amoxicillin, Cefaclor, Cefadroxil, Tetracyclin, Erythromycin, ein pharmazeutisch annehmbares Salz eines dieser Wirkstoffe oder eine Kombination zweier oder mehrerer dieser Wirkstoffe und Salze enthält.

8. Zusammensetzung nach einem der Ansprüche 1 bis 7, dadurch gekennzeichnet, dass sie als speichelflussförderndes Mittel eine wasserlösliche organische Säure bzw. ein wasserlösliches Salz einer wasserlöslichen organischen Säure und/oder eine wasserlösliche, osmotisch wirkende Substanz enthält.

9. Zusammensetzung nach einem der Ansprüche 1 bis 8, dadurch gekennzeichnet, dass sie als speichelflussförderndes Mittel Weinsäure, Zitronensäure, Apfelsäure, Ascorbinsäure, ein Natrium- oder Kaliumsalz dieser Säuren, Glucose, Fructose, Saccharose, Xylit, Mannit, Sorbit, Maltit oder eine Kombination zweier oder mehrerer dieser Verbindungen enthält.

10. Zusammensetzung nach einem der Ansprüche 1 bis 9, dadurch gekennzeichnet, dass der Überzug aus zwei oder mehreren Schichten besteht und die als 1 %-ige (Gew./Gew.) wässrige Lösung bei 25°C gemessene Viskosität des hydratisierbaren Polymers einer Schicht des Überzugs jeweils nicht grösser ist als die als 1 %-ige (Gew./Gew.) wässrige Lösung bei 25°C gemessene Viskosität des hydratisierbaren Polymers der benachbarten inneren Schicht des Überzugs.

11. Zusammensetzung nach Anspruch 10, dadurch gekennzeichnet, dass die äusserste Schicht des Überzugs ein hydratisierbares Polymer mit einer Viskosität von 25 bis 5000 mPa·s enthält und die zweitäusserste Schicht des Überzugs ein nicht-ionisches, hydratisierbares Polymer mit einer Viskosität von 5000 bis 10000 mPa·s und/oder ein ionisches, hydratisierbares Polymer mit einer Viskosität von 5000 bis 30000 mPa·s enthält, wobei sich die Viskositätswerte jeweils auf die bei 25°C gemessene Viskosität einer 1 %-igen (Gew./Gew.) wässrigen Lösung des Polymers beziehen.

12. Zusammensetzung nach Anspruch 10 oder 11, dadurch gekennzeichnet, dass die äusserste Schicht des Überzugs Polyvinylpyrrolidon oder einen Celluloseether mit einer Viskosität von 25 bis 5000 mPa·s und die zweitäusserste Schicht des Überzugs Natriumcarboxymethylcellulose mit einer Viskosität von 5000 bis 8000 mPa·s, Polyacrylsäure mit einer Viskosität von 5000 bis 30000 mPa·s oder einen Celluloseether mit einer Viskosität von 5000 bis 10000 mPa·s enthält, wobei sich die Viskositätswerte jeweils auf die bei 25°C gemessene Viskosität einer 1 %-igen (Gew./Gew.) wässrigen Lösung des Polymers beziehen.

13. Zusammensetzung nach einem der Ansprüche 10 bis 12, dadurch gekennzeichnet, dass in der zweitäussersten Schicht des Überzugs ein hydratisierbares Polymer mit einer mittleren Korngrösse von höchstens 50 µm verwendet wird.

14. Zusammensetzung nach einem der Ansprüche 10 bis 13, dadurch gekennzeichnet, dass die zweitäusserste Schicht des Überzugs in einer Menge von 0,25 bis 50 Gew.-%, berechnet als im wesentlichen wasserfreie Schicht und bezogen auf den im wesentlichen wasserfreien wirkstoffhaltigen Kern, und die äusserste Schicht des Überzugs in einer Menge von 3 bis 60 Gew.-%, berechnet als im wesentlichen wasserfreie Schicht und bezogen auf die im wesentlichen wasserfreie Zusammensetzung, vorliegt.

15. Zusammensetzung nach einem der Ansprüche 1 bis 14, dadurch gekennzeichnet, dass der Kern eine geschmackskaschierende, magensaftresistente oder die Wirkstofffreigabe verzögernde Lackschicht aufweist.

16. Zusammensetzung nach einem der Ansprüche 1 bis 15, dadurch gekennzeichnet, dass die überzogenen Teilchen einen maximalen Durchmesser von 0,25 bis 12 mm aufweisen.

17. Zusammensetzung nach einem der Ansprüche 1 bis 16, dadurch gekennzeichnet, dass sie mehrere überzogene Teilchen enthält und die bei Kontakt mit Speichel gebildete formbare Masse ein Zusammenkleben der Teilchen bewirkt.

18. Zusammensetzung nach einem der Ansprüche 1 bis 16, dadurch gekennzeichnet, dass sie aus einem einzigen überzogenen Teilchen besteht, das einen maximalen Durchmesser von 3 bis 12 mm aufweist.

19. Zusammensetzung nach einem der Ansprüche 1 bis 18, dadurch gekennzeichnet, dass sie im wesentlichen wasserfrei ist.

20. Zusammensetzung nach einem der Ansprüche 1 bis 17, dadurch gekennzeichnet, dass sie mehrere überzogene Teilchen und Wasser in einer Menge von 23 bis 75 Gew.-% enthält und als eine einzige zusammenhängende, viskose Masse mit ausreichender Konsistenz vorliegt, dass sie, ohne zu zerfallen, mit der Hand oder mittels eines Löffels oder Spatels eingenommen werden kann.

21. Verwendung eines hydratisierbaren, pharmazeutisch annehmbaren Polymers zur Herstellung einer pharmazeutischen Zusammensetzung, die bei Kontakt mit Speichel oder Wasser eine plastische Masse bildet und die ohne Flüssigkeit oder mit Wasser in einer Menge von bis zu 300 Gew.-%, bezogen auf die im wesentlichen wasserfreie Zusammensetzung, oral verabreicht werden kann, dadurch gekennzeichnet, dass man ein oder mehrere Teilchen, enthaltend mindestens einen pharmazeutischen Wirkstoff in wirksamer Menge, mit einer oder mehreren Schichten überzieht, wobei
(a) die Schicht bzw. die Schichten mindestens ein hydratisierbares, pharmazeutisch annehmbares Polymer enthalten, das bei Kontakt mit Speichel oder Wasser eine zusammenhängende, formbare, viskose, plastische, an der Oberfläche schlüpfrige, an der Mundschleimhaut nicht klebende Masse bildet, die das wirkstoffhaltige Teilchen bzw. die wirkstoffhaltigen Teilchen umhüllt und die den Austritt von wirkstoffhaltigen Teilchen aus der Masse und die Freisetzung von Wirkstoff in der Mundhöhle verhindert, und
(b) die Schicht bzw. die äusserste Schicht eine wirksame Menge mindestens eines speichelflussfördernden Mittels enthält,
dass man, gewünschtenfalls, die überzogenen Teilchen zusammen mit pharmazeutischen Hilfsstoffen in eine galenische Darreichungsform bringt und
dass man, gewünschtenfalls, die Zusammensetzung mit Wasser in einer Menge von bis zu 300 Gew.-%, bezogen auf die im wesentlichen wasserfreie Zusammensetzung, versetzt.

22. Pharmazeutische Zusammensetzung zur oralen Verabreichung, umfassend ein oder mehrere Teilchen, die mindestens einen pharmazeutischen Wirkstoff in wirksamer Menge enthalten, und eine durch Kontakt mit Speichel gebildete zusammenhängende, viskose, plastische, an der Oberfläche schlüpfrige, an der Mundschleimhaut nicht klebende Masse, die das wirkstoffhaltige Teilchen bzw. die wirkstoffhaltigen Teilchen umhüllt und die den Austritt von wirkstoffhaltigen Teilchen aus der Masse und die Freisetzung von Wirkstoff in der Mundhöhle verhindert, und die eine wirksame Menge mindestens eines speichelflussfördernden Mittels und mindestens ein hydratisierbares, pharmazeutisch annehmbares Polymer in mindestens teilweise hydratisierter Form enthält.

23. Arzneimittelpackung, umfassend eine pharmazeutische Zusammensetzung gemäss einem der Ansprüche 1 bis 20 und die Anweisung, die Zusammensetzung durch direkte Applikation in den Mund einzunehmen oder vor der Einnahme mit einer dosierten Menge von 30 bis 300 Gew.-% Wasser, bezogen auf die im wesentlichen wasserfreie pharmazeutische Zusammensetzung, zu versetzen.

## Claims

1. Pharmaceutical composition for oral administration, containing at least one pharmaceutically active ingredient in an effective amount and comprising one or more coated particles which have a core containing the at least one pharmaceutically active ingredient, and have a coating consisting of one or more layers, characterized in that
(a) the coating layer or the coating layers contain at least one hydratable, pharmaceutically acceptable polymer which, on contact with saliva or water, forms a coherent, mouldable, viscous, plastic mass which is slippery on the surface and does not adhere to the oral mucosa, which envelops the active ingredient-containing particle or the active ingredient-containing particles, and which prevents active ingredient-containing particles escaping from the mass, and release of active ingredient in the mouth, and
(b) the coating layer or the outermost of the coating layers contains an effective amount of at least one salivation-promoting agent.

2. Composition according to Claim 1, characterized in that it contains as hydratable polymer a nonionic polymer with a viscosity, measured as 1% strength (weight/weight) aqueous solution at 25°C, of from 3 to 10,000 mPa·s or an ionic polymer with a viscosity, measured as 1% strength (weight/weight) aqueous solution at 25°C, of from 3 to 30,000 mPa·s.

3. Composition according to Claim 1 or 2, characterized in that it contains as hydratable polymer methylcellulose, hydroxyethylcellulose, hydroxypropylcellulose, hydroxypropylmethylcellulose, polyvinylpyrrolidone, sodium carboxymethylcellulose, polyacrylic acid, polyacrylate, alginic acid, alginate, pectin, xanthan, galactomannan, guar gum, hydroxypropyl-guar gum, gelatin and/or gum arabic.

4. Composition according to any of Claims 1 to 3, characterized in that it contains a hydratable polymer with a viscosity, measured as 1% strength (weight/weight) aqueous solution at 25°C, of at least about 25 mPa·s.

5. Composition according to any of Claims 1 to 4 characterized in that the hydratable polymer has an average particle size not exceeding 200 µm.

6. Composition according to any of Claims 1 to 5, characterized in that the coating is present in an amount of from 5 to 75% by weight, based on the essentially anhydrous composition.

7. Composition according to any of Claims 1 to 6 characterized in that it contains as pharmaceutically active ingredient loperamide, mesalazine, olsalazine, cimetidine, ranitidine, famotidine, nizatidine, omeprazole, sucralfate, pantoprazole, pancreatin, bisacodyl, lactulose, acetylsalicylic acid, paracetamol, ibuprofen, morphine, tramadol, naproxen, diclofenac, piroxicam, terfenadine, astemizole, ambroxol, acetylcysteine, theophylline, atenolol, nifedipine, diltiazem, verapamil, isosorbide mononitrate, amitriptyline, nitrazepam, budesonide, ciprofloxacin, norfloxacin, ofloxacin, amoxicillin, cefaclor, cefadroxil, tetracycline, erythromycin, a pharmaceutically acceptable salt of one of these active ingredients or a combination of two or more of these active ingredients and salts.

8. Composition according to any of Claims 1 to 7, characterized in that it contains as salivation-promoting agent a water-soluble organic acid or a water-soluble salt of a water-soluble organic acid and/or a water-soluble, osmotically active substance.

9. Composition according to any of Claims 1 to 8, characterized in that it contains as salivation-promoting agent tartaric acid, citric acid, malic acid, ascorbic acid, a sodium or potassium salt of these acids, glucose, fructose, sucrose, xylitol, mannitol, sorbitol, maltitol or a combination of two or more of these compounds.

10. Composition according to any of Claims 1 to 9 characterized in that the coating consists of two or more layers, and the viscosity, measured as 1% strength (weight/weight) aqueous solution at 25°C, of the hydratable polymer in a layer of the coating is in each case no greater than the viscosity, measured as 1% strength (weight/weight) aqueous solution at 25°C, of the hydratable polymer in the adjacent inner layer of the coating.

11. Composition according to Claim 10, characterized in that the outermost layer of the coating contains a hydratable polymer with a viscosity of from 25 to 5000 mPa·s, and the second outermost layer of the coating contains a nonionic hydratable polymer with a viscosity of from 5000 to 10,000 mPa·s and/or an ionic hydratable polymer with a viscosity of from 5000 to 30,000 mPa·s, where the viscosities in each case relate to the viscosity of a 1% strength (weight-weight) aqueous solution of the polymer measured at 25°C.

12. Composition according to Claim 10 or 11, characterized in that the outermost layer of the coating contains polyvinylpyrrolidone or a cellulose ether with a viscosity of from 25 to 5000 mPa·s, and the second outermost layer of the coating contains sodium carboxymethylcellulose with a viscosity of from 5000 to 8000 mPa·s, polyacrylic acid with a viscosity of from 5000 to 30,000 mPa·s or a cellulose ether with a viscosity of from 5000 to 10,000 mPa·s, where the viscosities in each case relate to the viscosity of a 1% strength (weight/weight) aqueous solution of the polymer measured at 25°C.

13. Composition according to any of Claims 10 to 12, characterized in that a hydratable polymer with an average particle size not exceeding 50 µm is used in the second outermost layer of the coating.

14. Composition according to any of Claims 10 to 13, characterized in that the second outermost layer of the coating is present in an amount of from 0.25 to 50% by weight, calculated as essentially anhydrous layer and based on the essentially anhydrous active ingredient-containing core, and the outermost layer of the coating is present in an amount of from 3 to 60% by weight, calculated as essentially anhydrous layer and based on the essentially anhydrous composition.

15. Composition according to any of Claims 1 to 14, characterized in that the core has a taste-masking coating layer which is resistant to gastric fluid or delays the release of active ingredient.

16. Composition according to any of Claims 1 to 15, characterized in that the coated particles have a maximum diameter of from 0.25 to 12 mm.

17. Composition according to any of Claims 1 to 16, characterized in that it contains several coated particles, and the mouldable mass formed on contact with saliva causes the particles to stick together.

18. Composition according to any of Claims 1 to 16, characterized in that it consists of a single coated particle which has a maximum diameter of from 3 to 12 mm.

19. Composition according to any of Claims 1 to 18, characterized in that it is essentially anhydrous.

20. Composition according to any of Claims 1 to 17, characterized in that it contains several coated particles and water in an amount of from 23 to 75% by weight, and is in the form of a single, coherent, viscous mass with sufficient consistency to allow it to be taken, without disintegrating, by hand or using a spoon or spatula.

21. Use of a hydratable, pharmaceutically acceptable polymer for producing a pharmaceutical composition which forms a plastic mass on contact with saliva or water and which can be administered orally without liquid or with water in an amount of up to 300% by weight, based on the essentialy anhydrous composition, characterized
in that one or more particles containing at least one pharmaceutically active ingredient in an effective amount are coated with one or more layers, where
(a) the layer or layers contain at least one hydratable, pharmaceutically acceptable polymer which, on contact with saliva or water, forms a coherent, mouldable, viscous, plastic mass which is slippery on the surface and does not adhere to the oral mucosa, which envelops the active ingredient-containing particle or the active ingredient-containing particles, and which prevents active ingredient-containing particles escaping from the mass, and active ingredient being released in the mouth, and
(b) the layer or the outermost layer contains an effective amount of at least one salivation-promoting agent,
in that, if desired, the coated particles are converted with pharmaceutical ancillary substances into a pharmaceutical dosage form, and
in that, if desired, the composition is mixed with water in an amount of up to about 300% by weight, based on the essentially anhydrous composition.

22. Pharmaceutical composition for oral administration, comprising one or more particles which contain at least one pharmaceutically active ingredient in an effective amount, and a coherent, viscous, plastic mass which is formed by contact with saliva, is slippery on the surface and does not adhere to the oral mucosa, which envelops the active ingredient-containing particle or the active ingredient-containing particles, and which prevents active ingredient-containing particles escaping from the mass and active ingredient being released in the mouth, and which contains an effective amount of at least one salivation-promoting agent and at least one hydratable, pharmaceutically acceptable polymer in at least partly hydyrated form.

23. Medicinal product pack comprising a pharmaceutical composition according to any of Claims 1 to 20 and the instructions that the composition be taken by direct administration into the mouth or, before intake, be mixed with a metered amount of from 30 to 300% by weight of water, based on the essentially anhydrous pharmaceutical composition.

## Revendications

1. Composition pharmaceutique pour administration par voie orale, contenant au moins une substance active pharmaceutique en quantité efficace et comprenant une ou plusieurs particules revêtues qui présentent un noyau contenant au moins la substance active pharmaceutique et un revêtement consistant en une ou plusieurs couches, caractérisée en ce que
(a) la couche de revêtement ou les couches de revêtement contiennent au moins un polymère hydratable, pharmaceutiquement acceptable, qui au contact de salive ou d'eau, forme une masse agrégée, susceptible d'être formée, visqueuse, plastique, de surface glissante, qui ne colle pas à la muqueuse buccale, qui enveloppe la particule contenant la substance active ou les particules contenant la substance active et qui prévient la sortie de particules contenant la substance active hors de la masse et la mise en liberté de substance active dans la cavité buccale et
(b) la couche de revêtement ou la couche extérieure de revêtement contient une quantité efficace d'au moins un agent salivant.

2. Composition selon la revendication 1, caractérisée en ce qu'elle contient en tant que polymère hydratable un polymère non ionique dont la viscosité, mesurée pour une solution aqueuse à 1% (poids/poids) à 25°C, est de 3 à 10000 mPa·s ou un polymère ionique dont la viscosité, mesurée pour une solution aqueuse à 1% (poids/poids) à 25°C, est de 3 à 30000 mPa·s.

3. Composition selon la revendication 1 ou 2, caractérisée en ce qu'elle contient en tant que polymère hydratable la méthylcellulose, l'hydroxyéthylcellulose, l'hydroxypropylcellulose, l'hydroxypropylméthylcellulose, la polyvinylpyrrolidone, la carboxyméthylcellulose sodique, l'acide polyacrylique, un polyacrylate, l'acide alginique, un alginate, la pectine, la xanthane, la galactomannane, la résine de guar, la résine de guar hydroxypropylée, la gélatine et/ou la gomme arabique.

4. Composition selon l'une quelconque des revendications 1 à 3, caractérisée en ce qu'elle contient un polymère hydratable dont la viscosité, mesurée pour une solution aqueuse à 1% (poids/poids) à 25°C, est d'au moins environ 25 mPs·s.

5. Composition selon l'une quelconque des revendications 1 à 4, caractérisée en ce que le polymère hydratable présente une dimension corpusculaire moyenne d'au plus 200 µm.

6. Composition selon l'une quelconque des revendications 1 à 5, caractérisée en ce que le revêtement est présent en une quantité de 5 à 75% en poids par rapport à la composition sensiblement exempte d'eau.

7. Composition selon l'une quelconque des revendications 1 à 6, caractérisée en ce qu'elle contient en tant que substance active pharmaceutique le lopéramide, la mésalazine, l'olsalazine, la cimétidine, la ranitidine, la famotidine, la nizatidine, l'oméprazole, le sucralfate, le pantoprazole, la pancréatine, le bisacodyle, le lactulose, l'acide acétylsalicylique, le paracétamol, l'ibuprofène, la morphine, le tramadol, le naproxène, le diclofénac, le piroxicam, la terfénadine, l'astémizole, l'ambroxol, l'acétylcystéine, la théophylline, l'aténolol, la nifédipine, le diltiazem, le vérapamil, le mononitrate d'isosorbide, l'amitriptyline, le nitrazépam, le budésonide, la ciprofloxacine, la norfloxacine, l'ofloxacine, l'amoxicilline, le céfaclor, le céfadroxil, la tétracycline, l'érythromycine, un sel pharmaceutiquement acceptable de l'une de ces substances actives ou une association de deux ou plusieurs de ces substances actives et de ces sels.

8. Composition selon l'une quelconque des revendications 1 à 7, caractérisée en ce qu'elle contient en tant qu'agent salivant un acide organique soluble dans l'eau ou un sel soluble dans l'eau d'un acide organique soluble dans l'eau et/ou une substance soluble dans l'eau qui exerce un effet osmotique.

9. Composition selon l'une quelconque des revendications 1 à 8, caractérisée en ce qu'elle contient en tant qu'agent salivant l'acide tartrique, l'acide citrique, l'acide malique, l'acide ascorbique, un sel de sodium ou de potassium de ces acides, le glucose, le fructose, le saccharose, la xylite, la mannite, le sorbitol, le maltitol ou une association de deux ou plusieurs de ces composés.

10. Composition selon l'une quelconque des revendications 1 à 9, caractérisée en ce que le revêtement consiste en deux ou plusieurs couches et que la viscosité, mesurée pour une solution aqueuse à 1% (poids/poids) à 25°C, du polymère hydratable d'une couche du revêtement n'est en aucun cas supérieure à la viscosité, mesurée pour une solution aqueuse à 1% (poids/poids) à 25°C du polymère hydratable de la couche intérieure avoisinante du revêtement.

11. Composition selon la revendication 10, caractérisée en ce que la couche extérieure du revêtement contient un polymère hydratable dont la viscosité est de 25 à 5000 mPa·s et que la couche située sous la couche extérieure du revêtement contient un polymère non ionique hydratable dont la viscosité est de 5000 à 10000 mPa·s et/ou un polymère ionique hydratable dont la viscosité est de 5000 à 30000 mPa·s, étant entendu que les valeurs de la viscosité se rapportent chaque fois à la viscosité mesurée pour une solution aqueuse du polymère à 1% (poids/poids) à 25°C.

12. Composition selon la revendication 10 ou 11, caractérisée en ce que la couche extérieure du revêtement contient une polyvinylpyrrolidone ou un éther de cellulose dont la viscosité est de 25 à 5000 mPa·s et que la couche située sous la couche extérieure du revêtement contient une carboxyméthylcellulose sodique dont la viscosité est de 5000 à 8000 mPa·s, un acide polyacrylique dont la viscosité est de 5000 à 30000 mPa·s ou un éther de cellulose dont la viscosité est de 5000 à 10000 mPa·s, étant entendu que les valeurs de la viscosité se rapportent chaque fois à la viscosité mesurée pour une solution aqueuse du polymère à 1% (poids/poids) à 25°C.

13. Composition selon l'une quelconque des revendications 10 à 12, caractérisée en ce qu'est utilisé, dans la couche située sous la couche extérieure du revêtement, un polymère hydratable dont la dimension corpusculaire moyenne est d'au plus 50 µm.

14. Composition selon l'une quelconque des revendications 10 à 13, caractérisée en ce que la couche située sous la couche extérieure du revêtement est présente en une quantité de 0,25 à 50% en poids, calculée en tant que couche sensiblement exempte d'eau et par rapport au noyau sensiblement exempt d'eau contenant la substance active, et que la couche extérieure du revêtement est présente en une quantité de 3 à 60% en poids, calculée en tant que couche sensiblement exempte d'eau et par rapport à la composition sensiblement exempte d'eau.

15. Composition selon l'une quelconque des revendications 10 à 14, caractérisée en ce que le noyau présente une couche de laque masquant le goût, résistant au suc gastrique ou retardant la mise en liberté de la substance active.

16. Composition selon l'une quelconque des revendications 10 à 15, caractérisée en ce que les particules revêtues présentent un diamètre maximal de 0,25 à 12mm.

17. Composition selon l'une quelconque des revendications 1 à 16, caractérisée en ce qu'elle contient plusieurs particules revêtues et que la masse susceptible d'être formée, qui s'est formée au contact de la salive, provoque une agrégation des particules.

18. Composition selon l'une quelconque des revendications 1 à 16, caractérisée en ce qu'elle consiste en une seule particule revêtue dont le diamètre maximal est de 3 à 12mm.

19. Composition selon l'une quelconque des revendications 1 à 18, caractérisée en ce qu'elle est sensiblement exempte d'eau.

20. Composition selon l'une quelconque des revendications 1 à 17, caractérisée en ce qu'elle contient plusieurs particules revêtues et de l'eau en une quantité de 23 à 75% en poids et qu'elle se présente comme une seule masse agrégée, visqueuse, d'une consistance suffisante pour pouvoir être prise à la main ou à l'aide d'une cuillère ou d'une spatule sans se désagréger.

21. Utilisation d'un polymère hydratable, pharmaceutiquement acceptable, pour la préparation d'une composition pharmaceutique qui, au contact de salive ou d'eau, forme une masse plastique et qui peut être administrée par voie orale sans liquide ou avec de l'eau en une quantité allant jusqu'à 300% en poids, calculée par rapport à la composition sensiblement exempte d'eau, caractérisée en ce qu'on revêt une ou plusieurs particules, contenant au moins une substance active pharmaceutique en quantité efficace, d'une ou de plusieurs couches, étant entendu que
(a) la couche ou les couches contiennent au moins un polymère hydratable, pharmaceutiquement acceptable, qui au contact de salive ou d'eau forme une masse agrégée, susceptible d'être formée, visqueuse, plastique, de surface glissante, qui ne colle pas à la muqueuse buccale, qui enveloppe la particule contenant la substance active ou les particules contenant la substance active et qui prévient la sortie de particules contenant la substance active hors de la masse et la mise en liberté de substance active dans la cavité buccale, et que
(b) la couche ou la couche extérieure contient une quantité efficace d'au moins un agent salivant,
en ce que, si désiré, on met sous une forme d'administration galénique les particules revêtues ensemble avec des adjuvants pharmaceutiques et
en ce que, si désiré, on traite la composition par de l'eau en une quantité allant jusqu'à 300% en poids, calculée par rapport à la composition sensiblement exempte d'eau.

22. Composition pharmaceutique pour administration par voie orale, comprenant une ou plusieurs particules qui contiennent au moins une substance active pharmaceutique en quantité efficace et une masse agrégée, formée par contact avec la salive, visqueuse, plastique, de surface glissante, qui ne colle pas à la muqueuse buccale, qui enveloppe la particule contenant la substance active ou les particules contenant la substance active et qui prévient la sortie de particules contenant la substance active hors de la masse et la mise en liberté de substance active dans la cavité buccale, et qui contient une quantité efficace d'au moins un agent salivant et au moins un polymère hydratable, pharmaceutiquement acceptable, sous forme au moins partiellement hydratée.

23. Emballage de médicament, comprenant une composition pharmaceutique selon l'une quelconque des revendications 1 à 20 et les directives d'avoir à prendre la composition dans la bouche par application directe ou d'avoir à la traiter par de l'eau, avant la prise, en une quantité de 30 à 300% en poids par rapport à la composition pharmaceutique sensiblement exempte d'eau.
